(19) 

Europäisches
Patentamt
European
Patent Office
Office européen
des brevets

(11)  **EP 4 446 698 A1**

(12)  # EUROPEAN PATENT APPLICATION
published in accordance with Art. 153(4) EPC

(43) Date of publication:
**16.10.2024 Bulletin 2024/42**

(21) Application number: **22904309.6**

(22) Date of filing: **08.12.2022**

(51) International Patent Classification (IPC):
**G01B 21/00** *(2006.01)*  **G01B 21/22** *(2006.01)*

(52) Cooperative Patent Classification (CPC):
**G01B 21/00; G01B 21/22**

(86) International application number:
**PCT/JP2022/045370**

(87) International publication number:
**WO 2023/106382 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.12.2021 JP 2021201275**

(71) Applicant: **Kyocera Corporation
Kyoto-shi, Kyoto 612-8501 (JP)**

(72) Inventors:
• **KLINKIGT, Martin
Kyoto-shi, Kyoto 612-8501 (JP)**

• **KIMPARA, Hideyuki
Kyoto-shi, Kyoto 612-8501 (JP)**
• **MURAKAMI, Edwardo
Kyoto-shi, Kyoto 612-8501 (JP)**
• **SUZUKI, Takashi
Kyoto-shi, Kyoto 612-8501 (JP)**
• **KISHI, Masayuki
Kyoto-shi, Kyoto 612-8501 (JP)**
• **NAGATOMO, Takashi
Kyoto-shi, Kyoto 612-8501 (JP)**
• **NISHIDA, Naoki
Kyoto-shi, Kyoto 612-8501 (JP)**

(74) Representative: **Viering, Jentschura & Partner
mbB
Patent- und Rechtsanwälte
Am Brauhaus 8
01099 Dresden (DE)**

(54) **INFORMATION PROCESSING DEVICE, ELECTRONIC APPLIANCE, INFORMATION
PROCESSING SYSTEM, INFORMATION PROCESSING METHOD, AND PROGRAM**

(57)  An information processing device includes a controller. The controller is configured to acquire an estimated value of a posture angle of at least any one of multiple body parts of a user based on sensor data representing movement of at least some of the body parts of the user and a trained model. The trained model is trained and outputs the estimated value of the posture angle when input with the sensor data.

EP 4 446 698 A1

# FIG. 2

LOCAL COORDINATE SYSTEM

GLOBAL COORDINATE SYSTEM

**Description**

CROSS-REFERENCE TO RELATED APPLICATIONS

This application claims priority from Japanese Patent Application No. 2021-201275 filed in Japan on December 10, 2021, and the entire disclosure of this application is hereby incorporated by reference.

TECHNICAL FIELD

The present disclosure relates to an information processing device, an electronic device, an information processing system, an information processing method, and a program.

BACKGROUND OF INVENTION

Heretofore, a known technology detects movement of a user using a camera. For example, Patent Literature 1 describes a motion capture system that estimates the movement of an object based on images captured by multiple cameras.

CITATION LIST

PATENT LITERATURE

Patent Literature 1: Japanese Unexamined Patent Application Publication No. 2020-201183

SUMMARY

In an embodiment of the present disclosure, an information processing device includes a controller.
The controller is configured to acquire an estimated value of a posture angle of at least any one of multiple body parts of a user based on sensor data representing movement of at least some of the body parts of the user and a trained model.
The trained model is trained and outputs the estimated value of the posture angle when input with the sensor data.
In an embodiment of the present disclosure, an electronic device includes an output unit.
The output unit is configured to output data of the gait model generated by the information processing device.
In an embodiment of the present disclosure, an information processing system includes an information processing device.
The information processing device is configured to acquire an estimated value of a posture angle of at least any one of multiple body parts of a user based on sensor data representing movement of at least some of the body parts of the user and a trained model.
The trained model is trained and outputs the estimated value of the posture angle when input with the sensor data.
In an embodiment of the present disclosure, an information processing method includes
acquiring an estimated value of a posture angle of at least any one of multiple body parts of a user based on sensor data representing movement of at least some of the body parts of the user and a trained model.
The trained model is trained and outputs the estimated value of the posture angle when input with the sensor data.
In an embodiment of the present disclosure, a program is configured to cause a computer to execute
acquiring an estimated value of a posture angle of at least any one of multiple body parts of a user based on sensor data representing movement of at least some of the body parts of the user and a trained model.
The trained model is trained and outputs the estimated value of the posture angle when input with the sensor data.

BRIEF DESCRIPTION OF THE DRAWINGS

FIG. 1 is a diagram illustrating the schematic configuration of an information processing system according to an embodiment of the present disclosure.
FIG. 2 is a diagram for describing a local coordinate system and a global coordinate system.
FIG. 3 is a block diagram illustrating the configuration of the information processing system illustrated in FIG. 1.
FIG. 4 is a block diagram illustrating the configuration of a transformer.
FIG. 5 is a block diagram illustrating the configuration of "Multi-Head Attention".

FIG. 6 is a block diagram illustrating the configuration of "Scaled Dot-Product Attention".

FIG. 7 is a diagram illustrating examples of combinations of sensor data.

FIG. 8 is a graph of evaluation results.

FIG. 9 is a diagram illustrating subjects.

FIG. 10 is a graph of the posture angle of a subject's neck.

FIG. 11 is a graph of the posture angle of a subject's chest.

FIG. 12 is a graph of the posture angle of a subject's right upper arm.

FIG. 13 is a graph of the posture angle of a subject's left upper arm.

FIG. 14 is a graph of the posture angle of a subject's right forearm.

FIG. 15 is a graph of the posture angle of a subject's left forearm.

FIG. 16 is a graph of the posture angle of a subject's right thigh.

FIG. 17 is a graph of the posture angle of a subject's left thigh.

FIG. 18 is a graph of the posture angle of a subject's right lower leg.

FIG. 19 is a graph of the posture angle of a subject's left lower leg.

FIG. 20 is a graph of the posture angle of a subject's right foot.

FIG. 21 is a graph of the posture angle of a subject's left foot.

FIG. 22 is a graph of the posture angle of the right thigh of a subject evaluated as having a large center of gravity shift.

FIG. 23 is a graph of the posture angle of the right thigh of a subject evaluated as having a large center of gravity shift.

FIG. 24 is a graph of the posture angle of the right thigh of a subject evaluated as having a large center of gravity shift.

FIG. 25 is a graph of the posture angle of the right thigh of a subject evaluated as having a large center of gravity shift.

FIG. 26 is a graph of the posture angle of the right thigh of a subject evaluated as having a small center of gravity shift.

FIG. 27 is a graph of the posture angle of the right thigh of a subject evaluated as having a small center of gravity shift.

FIG. 28 is a graph of the posture angle of the right thigh of a subject evaluated as having a small center of gravity shift.

FIG. 29 is a graph of the posture angle of the right thigh of a subject evaluated as having a small center of gravity shift.

FIG. 30 is a graph of the posture angle of the right upper arm of a subject evaluated as having a large center of gravity shift.

FIG. 31 is a graph of the posture angle of the right upper arm of a subject evaluated as having a large center of gravity shift.

FIG. 32 is a graph of the posture angle of the right upper arm of a subject evaluated as having a large center of gravity shift.

FIG. 33 is a graph of the posture angle of the right upper arm of a subject evaluated as having a large center of gravity shift.

FIG. 34 is a graph of the posture angle of the right upper arm of a subject evaluated as having a small center of gravity shift.

FIG. 35 is a graph of the posture angle of the right upper arm of a subject evaluated as having a small center of gravity shift.

FIG. 36 is a graph of the posture angle of the right upper arm of a subject evaluated as having a small center of gravity shift.

FIG. 37 is a graph of the posture angle of the right upper arm of a subject evaluated as having a small center of gravity shift.

FIG. 38 is a flowchart illustrating operation of posture angle estimation processing performed by an electronic device illustrated in FIG. 1.

FIG. 39 is a block diagram illustrating the configuration of an information processing system according to another embodiment of the present disclosure.

FIG. 40 is a sequence diagram illustrating operation of estimation processing performed by the information processing system illustrated in FIG. 39.

## DESCRIPTION OF EMBODIMENTS

There is a demand to improve technologies for detection of user movement. For example, known technologies require the installation of cameras. According to an embodiment of the present disclosure, an improved technology for detecting user movement can be provided.

Embodiments of the present disclosure are described below while referring to the drawings. In the components illustrated in the drawings below, the same symbols are used for the same components.

(Configuration of System)

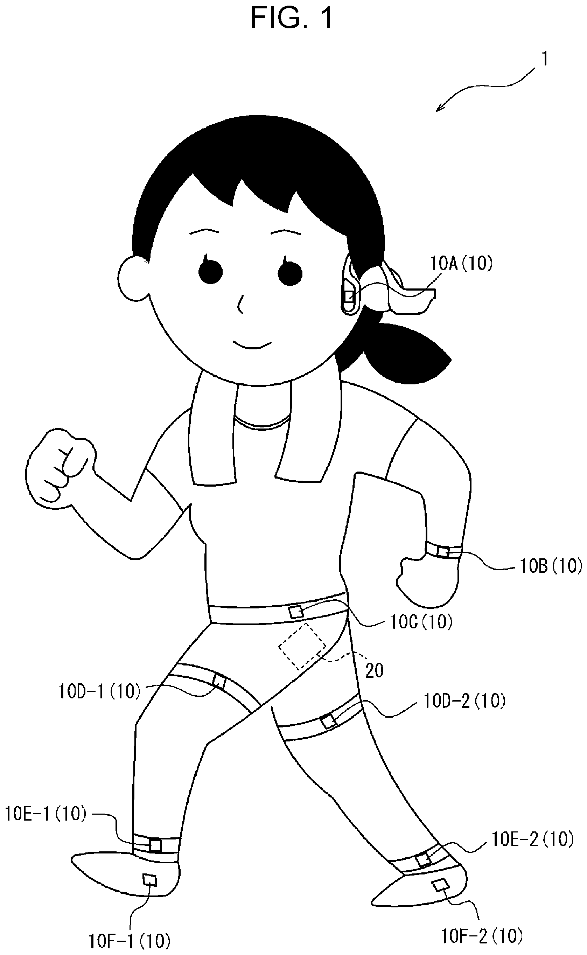

An information processing system 1, as illustrated in FIG. 1, can estimate the posture angle of any body part of a user who is performing periodic exercise. The information processing system 1 can generate a model, such as a 3D animation, illustrating the way in which a user is performing periodic exercise, for example, by estimating the posture angles of body parts across the user's entire body. The periodic exercise may be any exercise. For example, the periodic exercise may be walking, running, or pedaling a bicycle. In this embodiment, the periodic exercise is assumed to be walking. In other words, in this embodiment, the information processing system 1 is assumed to estimate the posture angles of the body parts of a user while walking. The user, for example, walks as exercise in his or her daily life.

The information processing system 1 includes a sensor device 10A, a sensor device 10B, a sensor device 10C, sensor devices 10D-1 and 10D-2, sensor devices 10E-1 and 10E-2, sensor devices 10F-1 and 10F-2, and an electronic device 20. However, the information processing system 1 does not need to include all of the sensor devices 10A, 10B, 10C, 10D-1, 10D-2, 10E-1, 10E-2, 10F-1, and 10F-1. The information processing system 1 only needs to include at least one selected from the group consisting of the sensor devices 10A, 10B, 10C, 10D-1, 10D-2, 10E-1, 10E-2, 10F-1, and 10F-1.

Hereafter, when the sensor devices 10D-1 and 10D-2 are not particularly distinguished from each other, the sensor devices 10D-1 and 10D-2 will be collectively referred to as the "sensor device 10D". When the sensor devices 10E-1 and 10E-2 are not particularly distinguished from each other, the sensor devices 10E-1 and 10E-2 will be collectively referred to as the "sensor device 10E". When the sensor devices 10F-1 and 10F-2 are not particularly distinguished from each other, the sensor devices 10F-1 and 10F-2 will be collectively referred to as the "sensor device 10F". When the sensor devices 10A to 10D are not particularly distinguished from each other, the sensor devices 10A to 10D will also be collectively referred to as the "sensor device 10".

The sensor device 10 and the electronic device 20 can communicate with each other via communication lines. The communication lines include at least one out of wired and wireless communication lines.

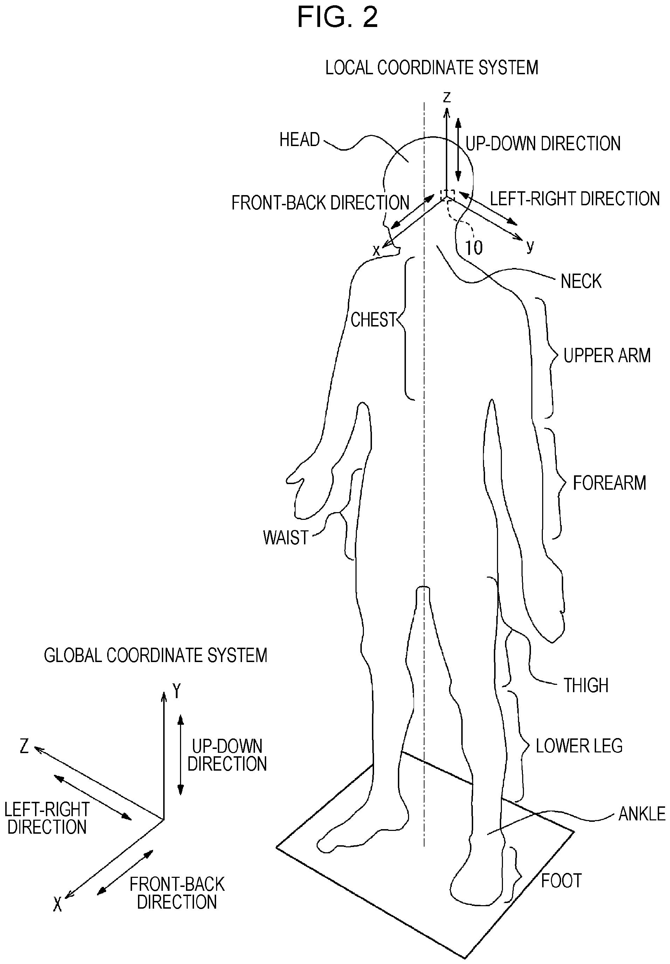

A local coordinate system is a coordinate system based on the positions of the sensor devices 10, as illustrated in FIG. 2. The position of the sensor device 10A is indicated in FIG. 2 with a dashed line as an example of the position of the sensor device 10. The local coordinate system consists of an x-axis, a y-axis, and a z-axis, for example. The x-axis, the y-axis, and the z-axis are orthogonal to one another. The x-axis is parallel to a front-back direction as viewed from the sensor device 10. The y-axis is parallel to a left-right direction as viewed from the sensor device 10. The z-axis is parallel to an up-down direction as viewed from the sensor device 10. The positive and negative directions of the x-axis, y-axis, and the z-axis may be set in accordance with the configuration and so forth of the information processing system 1.

A global coordinate system is a coordinate system based on the position of the user in the space in which the user walks, as illustrated in FIG. 2. The global coordinate system consists of an X-axis, a Y-axis, and a Z-axis, for example. The X-axis, the Y-axis, and the Z-axis are orthogonal to one another. The X-axis is parallel to a front-back direction as seen from the user's perspective. In this embodiment, the positive direction of the X-axis is assumed to be a direction from behind the user to in front of the user. The negative direction of the X-axis is assumed to be a direction from in front of the user to behind the user. The Y-axis is parallel to an up-down direction as seen from the user's perspective. In this embodiment, the positive direction of the Y-axis is assumed to be a direction from below the user to above the user. The negative direction of the Y-axis is assumed to be a direction from above the user to below the user. The Z-axis is a parallel to a left-right direction as seen from the user's perspective. In this embodiment, the positive direction of the Z-axis is assumed to be a direction from a region to the left of the user to a region to the right of the user. The negative direction of the Z-axis is assumed to be a direction from a region to the right of the user to a region to the left of the user. However, the positive and negative directions of the X-axis, Y-axis, and the Z-axis may be set in accordance with the configuration and so forth of the information processing system 1.

As illustrated in FIG. 1, the sensor device 10 is worn on at least some body parts of the user. The sensor device 10 detects sensor data representing the movement of the body part on which the sensor device 10 is worn. The sensor data is data of the local coordinate system. The sensor data is data representing the movement of at least some body parts of the user.

The sensor device 10A is worn on the user's head. For example, the sensor device 10A is worn on the user's ear. The sensor device 10A may be a wearable device. The sensor device 10A may be an earphone or may be contained in an earphone. Alternatively, the sensor device 10A may be a device that can be retrofitted to existing spectacles, earphones, or the like. The sensor device 10A may be worn on the user's head using any method. The sensor device 10A may be worn on the user's head by being incorporated into a hair accessory, such as a hair band or a hairpin, or an earring, a helmet, a hat, a hearing aid, a denture, or an implant.

The sensor device 10A may be worn on the user's head such that the x-axis of the local coordinate system based on the position of the sensor device 10A is parallel to the front-back direction of the head as seen from the user's perspective, the y-axis of the local coordinate system is parallel to the left-right direction of the head as seen from the user's perspective, and the z-axis of the local coordinate system is parallel to the up-down direction of the user's head as seen from the user's perspective. However, the x-axis, the y-axis, and the z-axis of the local coordinate system based on the position of the sensor device 10A do not necessarily need to be respectively aligned with the front-back direction, the left-right direction, and the up-down direction of the head as seen from the user's perspective. In this case, the relative orientation of the sensor device 10A with respect to the user's head may be initialized or identified as appropriate. The relative orientation may be initialized or identified by using information on the shape of a fixture used to attach the sensor device 10A to the user's head or by using captured image information of the user's head while the sensor device 10A is worn.

The sensor device 10A detects sensor data representing the movement of the user's head. The sensor data detected by the sensor device 10A includes, for example, at least any one of the following: the velocity of the user's head, the acceleration of the user's head, the angle of the user's head, the angular velocity of the user's head, the temperature of the user's head, and the geomagnetism at the position of the user's head.

The sensor device 10B is worn on the user's forearm. For example, the sensor device 10B is worn on the user's wrist. The sensor device 10B may be worn on the user's left forearm or may be worn on the user's right forearm. The sensor device 10B may be a wristwatch-type wearable device. The sensor device 10B may be worn on the user's forearm by using any method. The sensor device 10B may be worn on the user's forearm by being incorporated into a band, a bracelet, a friendship bracelet, a glove, a ring, an artificial fingernail, a prosthetic hand, and so on. The bracelet may be a bracelet worn by the user for a decorative purpose or may be a bracelet that allows the user to wear a key such as a locker key on his or her wrist.

The sensor device 10B may be worn on the user's forearm such that the x-axis of the local coordinate system based on the position of the sensor device 10B is parallel to the front-back direction of the wrist as seen from the user's perspective, the y-axis of the local coordinate system is parallel to the left-right direction of the wrist as seen from the user's perspective, and the z-axis of the local coordinate system is parallel to the rotational direction of the wrist as seen from the user's perspective. The rotational direction of the wrist is, for example, the direction in which the wrist twists and turns.

The sensor device 10B detects sensor data representing the movement of the user's forearm. For example, the sensor device 10B detects sensor data representing movement of the wrist. The sensor data detected by the sensor device 10B includes, for example, at least any of the following: the velocity of the user's forearm, the acceleration of the user's forearm, the angle of the user's forearm, the angular velocity of the user's forearm, the temperature of the user's forearm, and the geomagnetism at the position of the user's forearm.

The sensor device 10C is worn on the user's waist. The sensor device 10C may be a wearable device. The sensor device 10C may be worn on the user's waist by using a belt, a clip, or the like.

The sensor device 10C may be worn on the user's waist such that the x-axis of the local coordinate system based on the position of the sensor device 10C is aligned with the front-back direction of the waist as seen from the user's perspective, the y-axis of the local coordinate system is aligned with the left-right direction of the waist as seen from the user's perspective, and the z-axis of the local coordinate system is aligned with the rotational direction of the waist as seen from the user's perspective. The rotational direction of the waist is, for example, the direction in which the waist twists and turns.

The sensor device 10C detects sensor data representing the movement of the user's waist. The sensor data detected by the sensor device 10C includes, for example, at least any of the following: the velocity of the user's waist, the acceleration of the user's waist, the angle of the user's waist, the angular velocity of the user's waist, the temperature of the user's waist, and the geomagnetism at the position of the user's waist.

The sensor device 10D-1 is worn on the user's left thigh. The sensor device 10D-2 is worn on the user's right thigh. The sensor device 10D may be a wearable device. The sensor device 10D may be worn on the user's thigh by using any method. The sensor device 10D may be worn on the user's thigh using a belt, a clip, or the like. The sensor device 10D may be worn on the thigh by being placed in a pocket, which is in the vicinity of the thigh, of the pants worn by the user. The sensor device 10D may be worn on the user's thigh by being incorporated into pants, underwear, shorts, a supporter, a prosthetic leg, an implant, and so on.

The sensor device 10D may be worn on the user's thigh such that the x-axis of the local coordinate system based on the position of the sensor device 10D is parallel to the front-back direction of the thigh as seen from the user's perspective, the y-axis of the local coordinate system is parallel to the left-right direction of the thigh as seen from the user's perspective, and the z-axis of the local coordinate system is parallel to the rotational direction of the thigh as seen from the user's perspective. The rotational direction of the thigh is, for example, the direction in which the thigh twists and turns.

The sensor device 10D-1 detects sensor data representing the movement of the user's left thigh. The sensor device 10D-2 detects sensor data representing the movement of the user's right thigh. The sensor data detected by the sensor device 10D includes, for example, at least any of the following: the velocity of the user's thigh, the acceleration of the user's thigh, the angle of the user's thigh, the angular velocity of the user's thigh, the temperature of the user's thigh, and the geomagnetism at the position of the user's thigh.

The sensor device 10E-1 is worn on the user's left ankle. The sensor device 10E-2 is worn on the user's right ankle. The sensor device 10E may be a wearable device. The sensor device 10E may be worn on the user's ankle using any method. The sensor device 10E may be worn on the user's ankle using a belt, a clip, or the like. The sensor device 10E may be worn on the user's ankle by being incorporated into an anklet, a band, a friendship bracelet, a tattoo sticker, a supporter, a cast, a sock, a prosthetic leg, an implant, and so on.

The sensor device 10E may be worn on the user's ankle so that the x-axis of the local coordinate system based on the position of the sensor device 10E is aligned with the front-back direction of the ankle as seen from the user's perspective, the y-axis of the local coordinate system is aligned with the left-right direction of the ankle as seen from the user's perspective, and the z-axis of the local coordinate system is aligned with the rotational direction of the ankle as seen from the user's perspective. The rotational direction of the ankle is, for example, the direction in which the ankle twists and turns.

The sensor device 10E-1 detects sensor data representing the movement of the user's left ankle. The sensor device 10E-2 detects sensor data representing the movement of the user's right ankle. The sensor data detected by the sensor device 10E includes, for example, at least any of the following: the velocity of the user's ankle, the acceleration of the user's ankle, the angle of the user's ankle, the angular velocity of the user's ankle, the temperature of the user's ankle and the geomagnetism at the position of the user's ankle.

The sensor device 10F-1 is worn on the user's left foot. The sensor device 10F-2 is worn on the user's right foot. In this embodiment, the foot is the part extending from the user's ankle to the user's toes. The sensor device 10F may be a shoe-type wearable device. The sensor device 10F may be provided on or in a shoe. The sensor device 10F may be worn on the user's foot by using any method. The sensor device 10F may be worn on the user's foot by being incorporated into an anklet, a band, a friendship bracelet, an artificial fingernail, a tattoo sticker, a supporter, a cast, a sock, an insole, an artificial foot, a ring, an implant, and so on.

The sensor device 10F may be worn on the user's foot such that the x-axis of the local coordinate system based on the position of the sensor device 10F is parallel to the front-back direction of the foot as seen from the user's perspective, the y-axis of the local coordinate system is parallel to the left-right direction of the foot as seen from the user's perspective, and the z-axis of the local coordinate system is parallel to the up-down direction of the foot as seen from the user's perspective.

The sensor device 10F-1 detects sensor data representing the movement of the user's left foot. The sensor device 10F-2 detects sensor data representing the movement of the user's right ankle. The sensor data detected by the sensor device 10F includes, for example, at least any of the following: the velocity of the user's foot, the acceleration of the user's foot, the angle of the user's foot, the angular velocity of the user's foot, the temperature of the user's foot, and the geomagnetism at the position of the user's foot.

The electronic device 20 is carried by the user while walking, for example. The electronic device 20 is a mobile device such as a mobile phone, a smartphone, or a tablet.

In this embodiment, the electronic device 20 functions as an information processing device and acquires estimated values of the posture angles of body parts of the user based on sensor data detected by the sensor device 10 and a trained model described below. In this embodiment, the posture angle of a body part is the angle of the body part in the global coordinate system. Hereafter, in the posture angle of a body part, the angle at which the body part rotates around the X-axis is also referred to as a "posture angle $\theta X$". The angle at which the body part rotates around the Y-axis is also referred to as a "posture angle $\theta Y$". The angle at which the body part rotates around the Z-axis is also referred to as a "posture angle $\theta Z$". In this embodiment, the positive direction of the posture angle $\theta X$ is assumed to be the direction of clockwise rotation around the X axis when looking in the negative direction of the X axis. The negative direction of the posture angle $\theta X$ is assumed to be the direction of counterclockwise rotation around the X axis when looking in the negative direction of the X axis. The positive direction of the posture angle $\theta Y$ is assumed to be the direction of clockwise rotation around the Y axis when looking in the negative direction of the Y axis. The negative direction of the posture angle $\theta Y$ is assumed to be the direction of counterclockwise rotation around the Y axis when looking in the negative direction of the Y axis. The positive direction of the posture angle $\theta Z$ is assumed to be the direction of clockwise rotation around the Z axis when viewed in the negative direction of the Z axis. The negative direction of the posture angle $\theta Z$ is assumed to be the direction of counterclockwise rotation around the Z axis when looking in the negative direction of the Z axis.

[Configuration of Sensor Device]

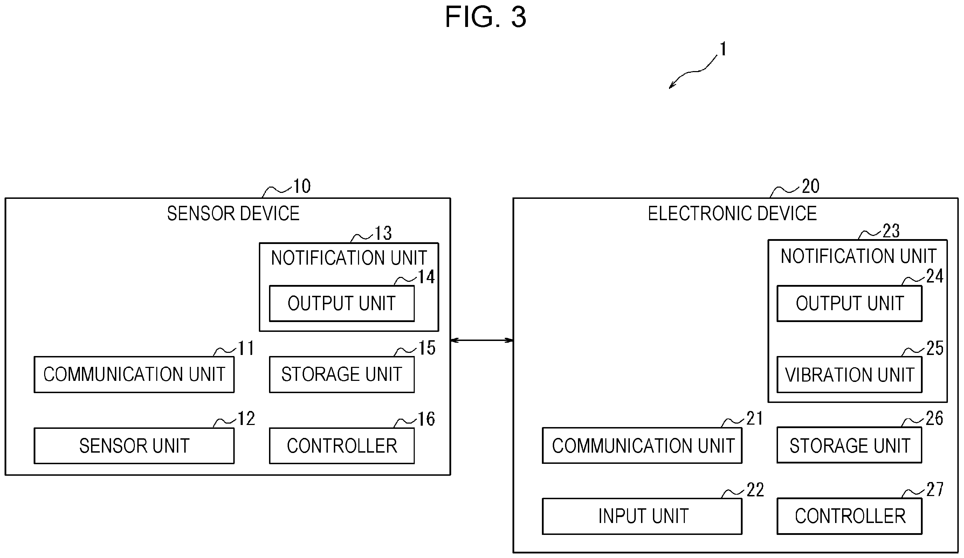

As illustrated in FIG. 3, the sensor device 10 includes a communication unit 11, a sensor unit 12, a notification unit 13, a storage unit 15, and a controller 16. However, the sensor devices 10C to 10F do not need to include the notification unit 13.

The communication unit 11 includes at least one communication module capable of communicating with the electronic device 20 via a communication line. The communication module is a communication module that is compatible with communication standards of communication lines. The communication line standards are short-range wireless communication standards including Bluetooth (registered trademark), infrared, and NFC (Near Field Communication), for example.

The sensor unit 12 includes any sensor depending on what sensor data is intended to be detected by the sensor device 10. The sensor unit 12 includes, for example, at least any one of the following: a three-axis motion sensor, a three-axis acceleration sensor, a three-axis velocity sensor, a three-axis gyro sensor, a three-axis magnetometer, a temperature sensor, an inertial measurement unit (IMU), and a camera. When the sensor unit 12 includes a camera, the camera can detect the movement of a body part of the user by analyzing images of the body part of the user captured by the camera.

When the sensor unit 12 includes an accelerometer and a magnetometer, data detected by each of the accelerometer and magnetometer may be used to calculate the initial angle of a body part to be detected by the sensor device 10. The data detected by each of the accelerometer and the magnetometer may be used to correct the data of the angle detected by the sensor device 10.

When the sensor unit 12 includes a gyro sensor, the angle of the body part to be detected by the sensor device 10 may be calculated by integrating the angular velocity detected by the gyro sensor over time.

The notification unit 13 reports information. In this embodiment, the notification unit 13 includes an output unit 14. However the notification unit 13 is not limited to the output unit 14. The notification unit 13 may include any component capable of reporting information.

The output unit 14 can output data. The output unit 14 includes at least one output interface capable of outputting data. The output interface is, for example, a display or a speaker. The display is, for example, an LCD (Liquid Crystal Display) or an organic EL (ElectroLuminescence) display.

If the output unit 14 is included in the sensor device 10A, the output unit 14 may include a speaker. If the output unit 14 is included in the sensor device 10B, the output unit 14 may include a display.

The storage unit 15 includes at least one semiconductor memory, at least one magnetic memory, at least one optical memory, or a combination of at least two of these types of memories. A semiconductor memory is, for example, a RAM (random access memory) or a ROM (read only memory). A RAM is for example, a SRAM (static random access memory) or a DRAM (dynamic random access memory). A ROM is, for example, an EEPROM (electrically erasable programmable read only memory). The storage unit 15 may function as a main storage device, an auxiliary storage device, or a cache memory. The storage unit 15 stores data used in operation of the sensor device 10 and data obtained by operation of the sensor device 10. For example, the storage unit 15 stores system programs, application programs, and embedded software.

The controller 16 includes at least one processor, at least one dedicated circuit, or a combination thereof. The processor can be a general-purpose processor such as a CPU (central processing unit) or a GPU (graphics processing unit), or a dedicated processor specialized for particular processing. A dedicated circuit is, for example, a FPGA (field-programmable gate array) or an ASIC (application specific integrated circuit). The controller 16 executes processing relating to operation of the sensor device 10 while controlling the various parts of the sensor device 10.

The controller 16 receives a signal instructing the start of data detection from the electronic device 20 via the communication unit 11. Upon receiving this signal, the controller 16 starts data detection. For example, the controller 16 acquires data detected by the sensor unit 12 from the sensor unit 12. The controller 16 transmits the acquired data, as sensor data, to the electronic device 20 via the communication unit 11. The signal instructing the start of data detection is transmitted from the electronic device 20 to multiple sensor devices 10 as a broadcast signal. A signal instructing the multiple sensor devices 10 to start data detection is transmitted as a broadcast signal to the multiple sensor devices 10 so that multiple sensor devices 10 can start data detection simultaneously.

The controller 16 acquires data from the sensor unit 12 at a preset time interval and transmits the acquired data as sensor data via the communication unit 11. The time interval may be set based on the walking speed of a typical user, for example. The same time interval may be used for each of the multiple sensor devices 10. This time interval being the same for the multiple sensor devices 10 allows the timings at which the multiple sensor devices 10 detect data to be synchronized.

[Configuration of Electronic Device]

As illustrated in FIG. 3, the electronic device 20 includes a communication unit 21, an input unit 22, a notification unit 23, a storage unit 26, and a controller 27.

The communication unit 21 includes at least one communication module capable of communicating with the sensor device 10 via a communication line. The communication module is at least one communication module that is compatible with communication standards of communication lines. The communication line standards are short-range wireless communication standards including, for example, Bluetooth (registered trademark), infrared, and NFC.

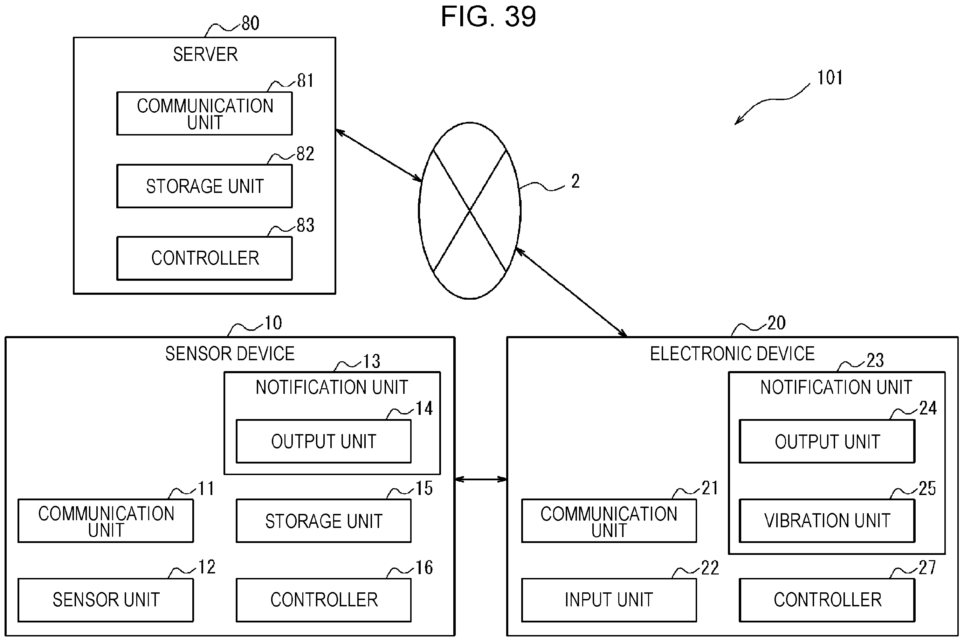

The communication unit 21 may further include at least one communication module that can connect to a network 2 as illustrated in FIG. 39 described later. The communication module is, for example, a communication module compatible with mobile communication standards such as LTE (Long Term Evolution), 4G (fourth Generation) or 5G (fifth Generation).

The input unit 22 can accept input from the user. The input unit 22 includes at least one input interface capable of accepting input from a user. The input interface takes the form of, for example, physical keys, capacitive keys, a pointing device, a touch screen integrated with the display, or a microphone.

The notification unit 23 reports information. In this embodiment, the notification unit 23 includes an output unit 24 and a vibration unit 25. However, the notification unit 23 is not limited to the output unit 24 and the vibration unit 25. The notification unit 23 may include any component capable of reporting information. The output unit 24 and vibration unit 25 may be mounted in the electronic device 20 or disposed in the vicinity of any of the sensor devices 10B to 10F.

The output unit 24 is capable of outputting data. The output unit 24 includes at least one output interface capable of outputting data. The output interface is, for example, a display or a speaker. The display is, for example, an LCD or organic EL display.

The vibration unit 25 is capable of making the electronic device 20 vibrate. The vibration unit 25 includes a vibration element. The vibration element is, for example, a piezoelectric element.

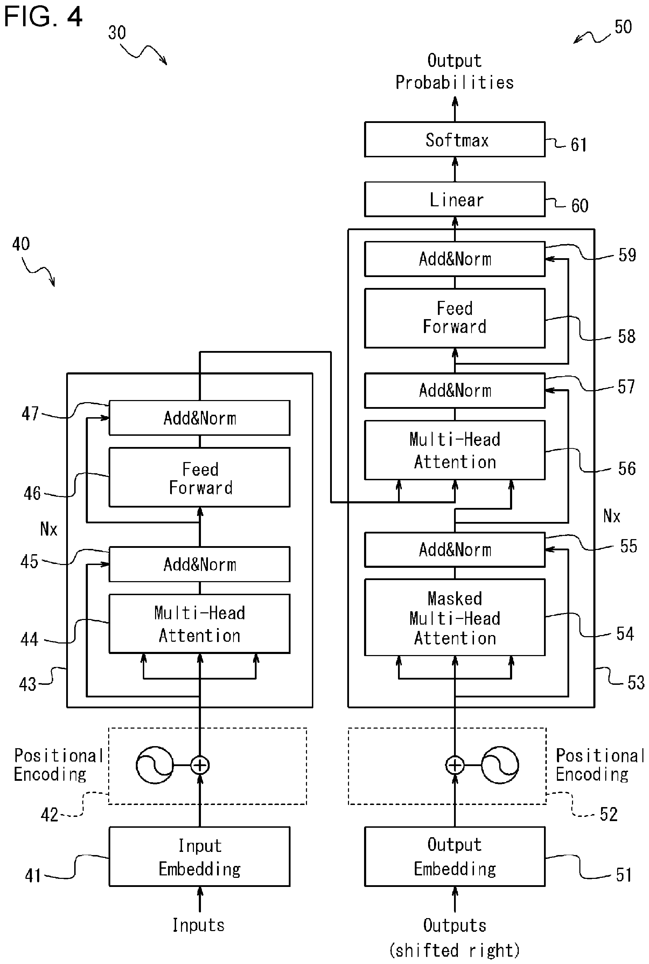

The storage unit 26 includes at least one semiconductor memory, at least one magnetic memory, at least one optical memory, or a combination of at least two of these types of memories. The semiconductor memory is, for example, a RAM or a ROM. The RAM is, for example, an SRAM or a DRAM. The ROM is, for example, an EEPROM. The storage unit 26 may function as a main storage device, an auxiliary storage device, or a cache memory. The storage unit 26 stores data used in operation of the electronic device 20 and data obtained by operation of the electronic device 20. For example, the storage unit 26 stores system programs, application programs, and embedded software. For example, the storage unit 26 stores data of a transformer 30 as illustrated in FIG. 4 described below and data used by the transformer 30.

The controller 27 includes at least one processor, at least one dedicated circuit, or a combination thereof. The processor can be a general-purpose processor such as a CPU or a GPU, or a dedicated processor specialized for particular processing. The dedicated circuit is, for example, an FPGA or an ASIC. The controller 27 executes processing relating to operation of the electronic device 20 while controlling the various parts of the electronic device 20. The controller 27 may perform the processing to be performed by the transformer 30 as illustrated in FIG. 4 described below.

The controller 27 accepts an input instructing execution of posture angle estimation processing via the input unit 22. This input is an input that causes the electronic device 20 to perform the estimation processing for the posture angles of the body parts of the user. This input is, for example, input from the input unit 22 by the user wearing the sensor device 10. The user inputs this input via the input unit 22, for example, before starting to walk. The controller 27 may accept an input indicating the user's height via the input unit 22, along with the input instructing execution of this estimation processing. Upon accepting the input indicating the user's height via the input unit 22, the controller 27 may store the accepted data of the user's height in the storage unit 26.

When the controller 27 accepts an input instructing execution of the posture angle estimation processing via the input unit 22, the controller 27 transmits a signal instructing the start of data detection as a broadcast signal to the multiple sensor devices 10 via the communication unit 21. After the signal instructing the start of data detection has been transmitted to the multiple sensor devices 10, sensor data is transmitted to the electronic device 20 from at least one of the sensor devices 10.

The controller 27 receives sensor data from at least one sensor device 10 via the communication unit 21. The controller 27 acquires the sensor data from the sensor device 10 by receiving the sensor data from the sensor device 10.

The controller 27 acquires an estimated value of the posture angle of at least any one of the multiple body parts of the user by using the sensor data and a trained model. When using sensor data of the global coordinate system, the controller 27 may acquire sensor data of the global coordinate system by performing a coordinate transformation on the sensor data of the local coordinate system acquired from the sensor device 10.

The trained model is, for example, generated by machine learning so as to output an estimated value of the posture angle of at least any one of the body parts of the user when input with sensor data. The body parts for which the trained model outputs estimated values may be set as appropriate in accordance with the application. In this embodiment, the controller 27 uses the transformer described in "Attention Is All You Need" by Ashish Vaswani et al, June 12, 2017, arXiv:1706.03762v5 [cs.CL]" as the trained model. The transformer can process time series data. The transformer will be described below with reference to FIG. 4. However, the trained model is not limited to a transformer. The controller 27 may use a trained model generated by machine learning based on any machine learning algorithm.

The controller 27 may acquire time series data of estimated values of the posture angles of body parts across the user's entire body by using the trained model. The controller 27 may generate a gait model using time-series data of estimated values of posture angles of body parts across the user's entire body and time-series data of the movement velocity of the user's waist. The movement velocity of the user's waist is a velocity in the global coordinate system. The controller 27 may acquire the time series data of the movement velocity of the user's waist by converting the sensor data detected by the sensor device 10C to data in the global coordinate system. Alternatively, the controller 27 may acquire time-series data of the movement velocity of the user's waist by using a trained model. When a transformer, which is described below, is used as a trained model, the controller 27 may acquire the normalized velocity, which is described below, of the user's waist from the transformer and multiply the acquired normalized velocity by the user's height in order to calculate data of the movement velocity of the user's waist. Here, the gait model to be generated is a model representing the way in which the user walks. The controller 27 may generate the gait model as a 3D animation. The controller 27 may generate the gait model by scaling a human model having a preset size using the height of the user. The body parts across the user's entire body to be used to generate the gait model may be set as appropriate centered on the waist. For example, the body parts across the user's entire body to be used to generate the gait model include the user's head, neck, chest, lumbar spine, pelvis, right and left thighs, right and left lower legs, right and left feet, right and left upper arms, and right and left forearms, as illustrated in FIG. 2. However the body parts across the user's entire body may be set as appropriate.

The controller 27 may cause the output unit 24 to output the generated gait model data. When the output unit 24 includes a display, the controller 27 may display the generated gait model on the display of the output unit 24. This configuration allows the user to grasp how he or she is walking.

The controller 27 may display the generated gait model as a 3D animation on the display of the output unit 24. In this case, the controller 27 may display the 3D animation of the gait model as a free viewpoint image on the display of the output unit 24 based on a user input accepted by the input unit 22. This configuration allows the user to grasp how he or she is walking in detail.

The controller 27 may transmit data of the estimated values of the user's body parts acquired using the trained model or data of the generated gait model to an external device via the network 2 as illustrated in FIG. 39 described below by using the communication unit 21. For example, the user may be receiving instruction from an instructor regarding his or her gait. In this case, the data of the estimated values of the user's body parts or the data of the gait model is transmitted to the external device, and this allows the instructor to monitor the way in which the user is walking via the external device and give the user instructions regarding his or her gait. Alternatively, the user may be an instructor. In this case, the data of the estimated values of the body parts of the user, who is an instructor, or the gait model is transmitted to an external device, and this allows a student to watch the way in which the instructor walks as a model via the external device.

[Configuration of Transformer]

The transformer 30, as illustrated in FIG. 4, can be trained to output a time series of estimated values of the posture angles of preset body parts of a user when sensor data along multiple time series are input. The transformer 30 can also be trained to output time-series data of the normalized velocity of the waist in addition to time-series data of the estimated values of the posture angles of the user's body parts. The normalized velocity of the user's waist is obtained by dividing the movement velocity of the user's waist by the user's height. The time range and time interval of the sensor data in the time series input to the transformer 30 may be set in accordance with the desired estimation accuracy and so on.

As illustrated in FIG. 4, the transformer 30 includes an encoder 40 and a decoder 50. The encoder 40 includes a functional unit 41, a functional unit 42, and an N-stage layer 43. The layer 43 includes a functional unit 44, a functional unit 45, a functional unit 46, and a functional unit 47. The decoder 50 includes a functional unit 51, a functional unit 52, an N-stage layer 53, a functional unit 60, and a functional unit 61. The layer 53 includes a functional unit 54, a functional unit 55, a functional unit 56, a functional unit 57, a functional unit 58, and a functional unit 59. The number of stages of the layer 43 included in the encoder 40 and the number of stages of the layer 53 included the decoder 50 are identical, i.e., N stages (N is a natural number).

The functional unit 41 is also referred to as "Input Embedding". An array of sensor data along multiple time series is input to the functional unit 41. For example, if the sensor data at time $t_i$ ($0 \leq i \leq n$) is denoted as "$D_{ti}$", the array of sensor data input to the functional unit 41 is represented as ($D_{t0}$, $D_{t1}$,..., $D_{tn}$). An array consisting of multiple types of sensor data may be input to the functional unit 41. For example, if two different pieces of sensor data at time $t_i$ ($0 \leq i \leq n$) are denoted as "$Dat;$" and "$Dbt;$" respectively, the array of sensor data input to the functional unit 41 is represented as ($Da_{t0}$, $Da_{t1}$,..., $Da_{tn}$, $Db_{t0}$, $Db_{t1}$,..., $Db_{tn}$).

The functional unit 41 converts each element of the array of input sensor data into a multidimensional vector, and in this way, generates a distributed representation vector. The number of dimensions of the multidimensional vector may be set in advance.

The functional unit 42 is also referred to as "Positional Encoding". The functional unit 42 assigns position information to the distributed representation vector.

The functional unit 42 calculates and adds position information to each element of the distributed representation vector. The position information represents the position of each element of the distributed representation vector in the array of sensor data input to the functional unit 41 and represents the position in the element array of the distributed representation vector. The functional unit 42 calculates position information PE of the ($2 \times i$)th element in the array of elements of the distributed representation vector using Eq. (1). The functional unit 42 calculates position information PE

of the $(2 \times i + 1)$th element in the array of elements of the distributed representation vector using Eq. (2).
[Math 1]

$$PE_{(pos,2i)} = sin(pos/10000^{2i/d_{model}})$$
Eq. (1)

[Math 2]

$$PE_{(pos,2i+1)} = cos(pos/10000^{2i/d_{model}})$$
Eq. (2)

In Eq. (1) and Eq. (2), pos is the position, in the array of sensor data input to the functional unit 41, of the elements of the distributed representation vector. dmodel is the number of dimensions of the distributed representation vector.

In the N-stage layer 43, a vector assigned with position information and having a distributed representation is input from the functional unit 42 to the first stage of the layer 43. The second and subsequent stages of the layer 43 are input with vectors from the previous stages of the layer 43.

The functional unit 44 is also referred to as "Multi-Head Attention". A Q (Query) vector, a K (Key) vector, and a V (Value) vector are input to the functional unit 44. The Q vector is obtained by multiplying the vector input to the layer 43 by a weight matrix WQ. The K vector is obtained by multiplying the vector input to the layer 43 by a weight matrix WK. The V vector is obtained by multiplying the vector input to layer 43 by a weight matrix WV. The transformer 30 learns the weight matrix WQ, the weight matrix WK, and the weight matrix WV during training.

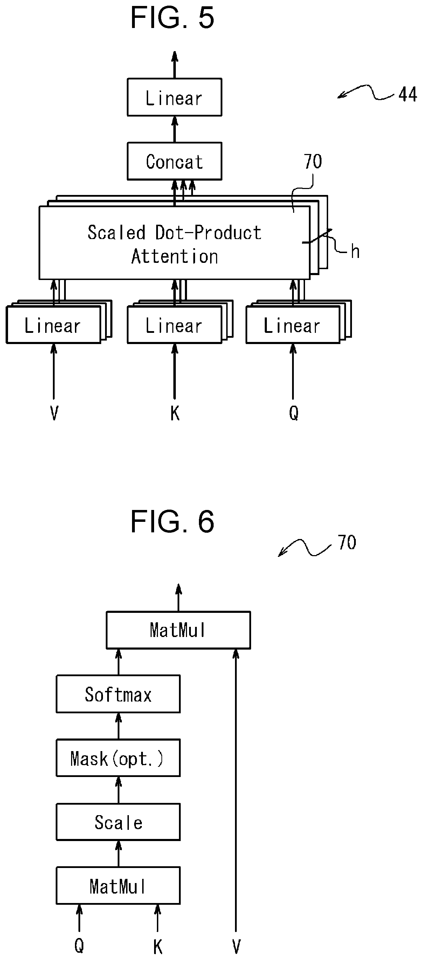

The functional unit 44 includes h functional units 70 and functional units "Linear" and "Contact" as illustrated in FIG. 5. The functional units 70 are also referred to as "Scaled Dot-Product Attention". The Q-vector, K-vector, and V-vector, divided into h pieces, are input to the functional units 70.

Each functional unit 70 includes functional units "MatMul", "Scale", "Mask (opt.)", and "Softmax", as illustrated in FIG. 6. The functional unit 70 calculates the Scaled Dot-Product Attention using the Q-vector, K-vector, and V-vector and Eq. (3).
[Math 3]

$$Attention(Q,K,V) = softmax(\frac{QK^T}{\sqrt{d_k}})V$$
Eq. (3)

In Eq. (3), dk is the number of dimensions of the Q vector and the K vector.

The functional unit 44 calculates Multi-Head Attention when the Scaled Dot-Product Attention is calculated by the h functional units 70 as illustrated in FIG. 5. The functional unit 44 calculates the Multi-Head Attention using Eq. (4).
[Math 4]

$$MutiHead(Q,K,V) = Concat(head_1, \ldots, head_h)W^O$$
Eq. (4)

Here,
$$head_i = Attention(QW_i^Q, KW_i^K, VW_i^V)$$

$$W_i^Q \in \mathbb{R}^{d_{model} \times d_k}, W_i^K \in \mathbb{R}^{d_{model} \times d_k}, W_i^V \in \mathbb{R}^{d_{model} \times d_v}, W^O \in \mathbb{R}^{hd_v \times d_{model}}$$

In Eq. (4), dk is the number of dimensions of the Q vector and the K vector. dv is the number of dimensions of the V vector.

The Multi-Head Attention calculated by the functional unit 44 is input to functional unit 45 as illustrated in FIG. 4.

The functional unit 45 is also referred to as "Add & Norm". The functional unit 45 adds the Multi-Head Attention calculated by the functional unit 44 to the vector input to the layer 43 and normalizes the resulting vector. The functional unit 45 inputs the normalized vector to the functional unit 46.

The functional unit 46 is also referred to as "Position-wise Feed-Forward Networks". The functional unit 46 generates an output by using an activation function such as a ReLU (Rectified Linear Unit) and a vector input from the functional unit 45. The functional unit 46 uses a different FFN (Feed-Forward Network) for each position in the element array of the sensor data along the time series before vectorization, i.e., the sensor data along the time series input to the functional unit 41. If the vector input from the functional unit 45 to the functional unit 46 is denoted as "x", then the functional unit 46 generates an output FFN(x) according to Eq. (5).
[Math 5]

$$FFN(x) = \max(0, xW_1 + b_1)W_2 + b_2 \qquad Eq. 5$$

In Eq. (5), W1 and W2 are coefficients, b1 and b2 are biases. W1 and W2 and b1 and b2 may be different for each position in the element array of sensor data along the time series before vectorization.

The functional unit 47 is also referred to as "Add & Norm". The functional unit 47 adds an output generated by the functional unit 46 to the vector output from the functional unit 45 and normalizes the resulting vector.

The functional unit 51 is also referred to as "Input Embedding". The functional unit 51 is input with the time series data of the estimated values of the posture angles of the body parts output by the decoder 50 in processing up to the immediately previous processing. When the decoder 50 estimates data of the posture angle etc. of a body part for the first time, the functional unit 51 may be input with preset data such as dummy data. In the same or a similar manner to the functional unit 41, the functional unit 51 converts each element of the input time series data into a multidimensional vector, and thereby generates a distributed representation vector. In the same or a similar manner to the functional unit 41, the number of dimensions of the multidimensional vector may be predetermined.

The functional unit 52 is also referred to as "Positional Encoding". The functional unit 52 assigns position information to the distributed representation vector in the same or a similar manner to the functional unit 42. In other words, the functional unit 52 calculates and adds position information for each element of the distributed representation vector. The position information represents the position of each element of the distributed representation vector in the array of time series data input to the functional unit 51 and in the element array of the distributed representation vector.

In the N-stage layer 53, a vector assigned with position information and having a distributed representation is input from the functional unit 52 to the first stage of the layer 53. The second and subsequent stages of the layer 53 are input with vectors from the previous stage of the layer 53.

The functional unit 54 is also referred to as "Masked Multi-Head Attention". The Q-vector, the K-vector, and the V-vector are input to the functional unit 54 in an identical or similar manner to the functional unit 44. The Q vector, the K vector, and the V vector are obtained by multiplying vectors input to the layer 53 by the same weight matrix or different weight matrices. The transformer 30 learns these weight matrices during training. The functional unit 54 calculates the Multi-Head Attention using the input Q-vector, K-vector, and V-vector, in the same or a similar manner to the functional unit 44.

Here, the functional unit 54 is input, in one go, with time series data of the posture angle etc. of a body part, which is the correct solution, during training of the transformer 30. During training of the transformer 30, the functional unit 54 masks the data, in the time series data of the posture angle etc. of the body part, at times from the time at which estimation is to be performed by the decoder 50.

The functional unit 55 is also referred to as "Add & Norm". The functional unit 55 adds the Multi-Head Attention calculated by the functional unit 54 to the vector input to the layer 53 and normalizes the resulting vector.

The functional unit 56 is also referred to as "Multi-Head Attention". A Q-vector, a K-vector, and a V-vector are input to the functional unit 56. The Q vector is the vector input to the functional unit 56 by the functional unit 55 after normalization. The K-vector and the V-vector are obtained by multiplying a vector output from the final stage of the layer 43 of the encoder 40 by the same or different weight matrices. The functional unit 56 calculates the Multi-Head Attention using the input Q-vector, K-vector, and V-vector, in the same or a similar manner to the functional unit 44.

The functional unit 57 is also referred to as "Add & Norm". The functional unit 57 adds the Multi-Head Attention calculated by the functional unit 56 to the vector output by the functional unit 55 and normalizes the resulting vector.

The functional unit 58 is also referred to as "Position-wise Feed-Forward Networks". The functional unit 58 generates an output by using an activation function such as ReLU and a vector input from the functional unit 57 in an identical or similar manner to the functional unit 46.

The functional unit 59 is also referred to as "Add & Norm". The functional unit 59 adds an output generated by the functional unit 58 to the vector output from the functional unit 57 and normalizes the resulting vector.

The functional unit 60 is also referred to as "Linear". The functional unit 61 is also referred to as "SoftMax". The output of the final stage of the layer 53 is output from the decoder 50 as data of the estimated value of the posture angle etc. of the body part after being normalized and so on by the functional unit 60 and the functional unit 61.

Here, the velocity of walking varies depending on the user. In other words, the gait cycle varies depending on the user. The gait cycle is the period of time from when one of the user's two feet lands on the ground or another surface until when that foot lands on the ground or the other surface again. Even through the gait cycle differs depending on the user, provided that there is time-series data of the sensor data for around half the average value of the gait cycle, the characteristics of the user's gait will appear in the time-series data of the sensor data. The transformer 30 can learn which part of the gait cycle the input sensor data corresponds to even if the time series data of the input sensor data is data of only part of the gait cycle. Therefore, the transformer 30 can be trained to output time series data of estimated values of posture angles when time-series data of sensor data for around half the average value of the gait cycle is input.

[Combinations of Sensor Data]

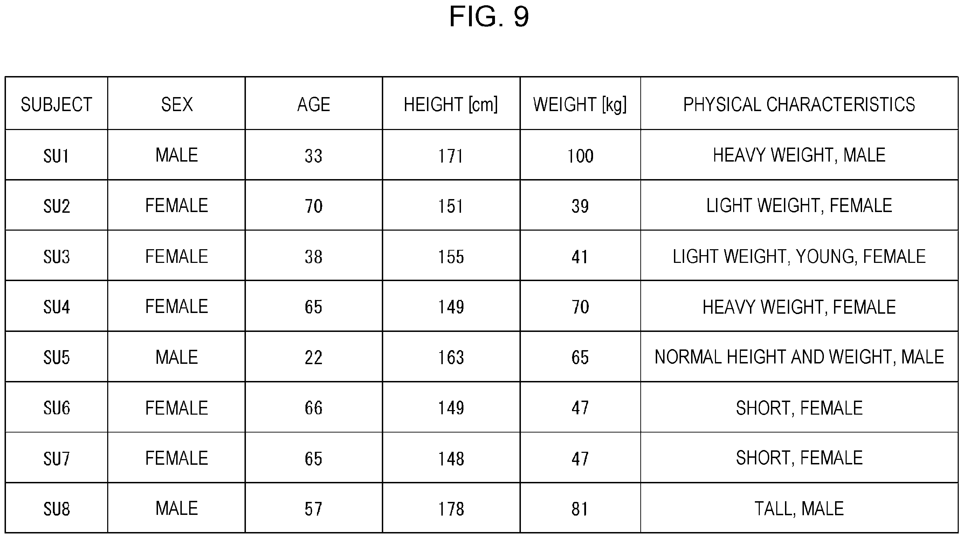

The controller 27 may use a transformer trained on one type of sensor data or a transformer trained on a combination of multiple types of sensor data. Combinations of multiple types of sensor data are, for example, cases C1, C2, C3, C4, C5, C6, C7, C8, C9, C10, C11, C12, and C13, as illustrated in FIG. 9.

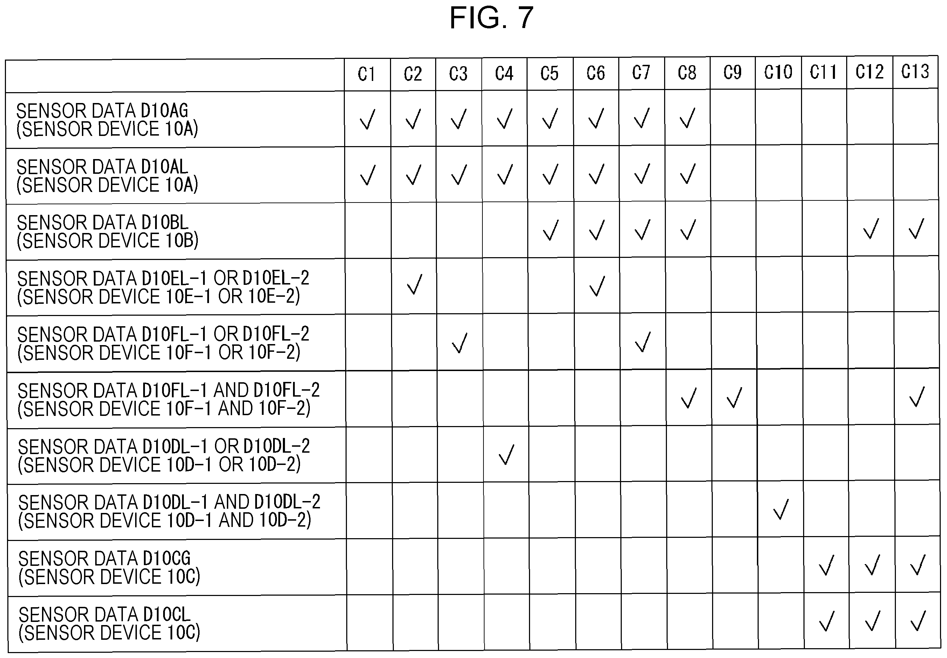

FIG. 7 illustrates examples of combinations of sensor data. The cases C1 to C13 are examples of combinations of sensor data. The controller 27 may select any of the cases C1 to C13 in accordance with the type of sensor device 10 that transmitted the sensor data to the electronic device 20. The data of the transformer 30 used in the cases C1 to C13 may be stored in storage unit 26 in association with the cases C1 to C13, respectively. The controller 27 acquires estimated values of the user's body parts by inputting the sensor data of the selected any one of the cases C1 to C13 to the transformer 30 corresponding to the selected any one of the cases C1 to C13.

<Case C1>

The controller 27 may select the case C1 if the sensor devices 10 that transmitted sensor data to the electronic device 20 include the sensor device 10A.

In the case C1, sensor data representing the movement of the user's head is used. In the case C1, sensor data D10AG and sensor data D10AL are used.

The sensor data D10AG is sensor data representing the movement of the user's head in the global coordinate system. The sensor data D10AG includes velocity data and acceleration data of the user's head with respect to the X-axis, velocity data and acceleration data of the user's head with respect to the Y-axis, and velocity data and acceleration data of the user's head with respect to the Z-axis in the global coordinate system. The controller 27 acquires the sensor data D10AG by performing a coordinate transformation on the sensor data in the local coordinate system acquired from the sensor device 10A.

The sensor data D10AL is sensor data representing the movement of the user's head in the local coordinate system with respect to the position of the sensor device 10A. The sensor data D10AL includes velocity data and acceleration data of the user's head with respect to the x-axis, velocity data and acceleration data of the user's head on the y-axis, and velocity data and acceleration data of the user's head with respect to the z-axis in the local coordinate system. The controller 27 acquires the sensor data D10AL from the sensor device 10A.

<Case C2>

The controller 27 may select the case C2 if the sensor devices 10 that transmitted sensor data to the electronic device 20 include the sensor device 10A and the sensor device 10E-1 or the sensor device 10E-2.

In the case C2, sensor data representing the movement of the user's head and sensor data representing the movement of either one of the user's two ankles are used. In the case C2, the sensor data D10AG, the sensor data D10AL, and sensor data D10EL-1 or sensor data D10EL-2 are used.

The sensor data D10EL-1 is sensor data representing the movement of the user's left ankle in the local coordinate system with respect to the position of the sensor device 10E-1. The sensor data D10EL-1 includes velocity data and acceleration data of the user's left ankle with respect to the x-axis, velocity data and acceleration data of the user's left ankle with respect to the y-axis, and velocity data and acceleration data of the user's left ankle with respect to the z-axis in the local coordinate system. The controller 27 acquires the sensor data D10EL-1 from the sensor device 10E-1.

The sensor data D10EL-2 is sensor data representing the movement of the user's right ankle in the local coordinate system with respect to the position of the sensor device 10E-2. The sensor data D10EL-2 includes velocity data and acceleration data of the user's right ankle with respect to the x-axis, velocity data and acceleration data of the user's right ankle with respect to the y-axis, and velocity data and acceleration data of the user's right ankle with respect to the z-axis in the local coordinate system. The controller 27 acquires the sensor data D10EL-2 from the sensor device 10E-2.

<Case C3>

The controller 27 may select the case C3 if the sensor devices 10 that transmitted sensor data to the electronic device 20 include the sensor device 10A and the sensor device 10F-1 or the sensor device 10F-2.

In the case C3, sensor data representing the movement of the user's head and sensor data representing the movement of either one of the user's two feet are used. In the case C3, the sensor data D10AG, the sensor data D10AL, and sensor data D10FL-1 or sensor data D10FL-2 are used.

The sensor data D10FL-1 is sensor data representing the movement of the user's left foot in the local coordinate system with respect to the position of the sensor device 10F-1. The sensor data D10FL-1 includes velocity data and acceleration data of the user's left foot with respect to the x-axis, velocity data and acceleration data of the user's left foot with respect to the y-axis, and velocity data and acceleration data of the user's left foot with respect to the z-axis in the local coordinate system. The controller 27 acquires the sensor data D10FL-1 from the sensor device 10F-1.

The sensor data D10FL-2 is sensor data representing the movement of the user's right foot in the local coordinate system with respect to the position of the sensor device 10F-2. The sensor data D10FL-2 includes velocity data and acceleration data of the user's right foot with respect to the x-axis, velocity data and acceleration data of the user's right foot with respect to the y-axis, and velocity data and acceleration data of the user's right foot with respect to the z-axis in the local coordinate system. The controller 27 acquires the sensor data D10FL-2 from the sensor device 10F-2.

<Case C4>

The controller 27 may select the case C4 if the sensor devices 10 that transmitted sensor data to the electronic device 20 include the sensor device 10A and the sensor device 10D-1 or the sensor device 10D-2.

In the case C4, sensor data representing the movement of the user's head and sensor data representing the movement of either one of the user's two thighs are used. In the case C4, the sensor data D10AG, the sensor data D10AL, and sensor data D10DL-1 or sensor data D10DL-2 are used.

The sensor data D10DL-1 is sensor data representing the movement of the user's left thigh in the local coordinate system with respect to the position of the sensor device 10D-1. The sensor data D10DL-1 includes velocity data and acceleration data of the user's left thigh with respect to the x-axis, velocity data and acceleration data of the user's left thigh with respect to the y-axis, and velocity data and acceleration data of the user's left thigh with respect to the z-axis in the local coordinate system. The controller 27 acquires the sensor data D10DL-1 from the sensor device 10D-1.

The sensor data D10DL-2 is sensor data representing the movement of the user's right thigh in the local coordinate system with respect to the position of the sensor device 10D-2. The sensor data D10DL-2 includes velocity data and acceleration data of the user's right thigh with respect to the x-axis, velocity data and acceleration data of the user's right thigh with respect to the y-axis, and velocity data and acceleration data of the user's right thigh with respect to the z-axis in the local coordinate system. The controller 27 acquires the sensor data D10DL-2 from the sensor device 10D-2.

<Case C5>

The controller 27 may select the case C5 if the sensor devices 10 that transmitted sensor data to the electronic device 20 include the sensor device 10A and the sensor device 10B.

In the case C5, sensor data representing the movement of the user's head and sensor data representing the movement of either one of the user's two wrists are used. In the case C5, the sensor data D10AG, the sensor data D10AL, and sensor data D10BL are used.

The sensor data D10BL is sensor data representing the movement of the user's wrist in the local coordinate system with respect to the position of the sensor device 10B. In this embodiment, the sensor data D10BL is assumed to be sensor data representing the movement of the user's left wrist. However, the sensor data D10BL may be sensor data representing the movement of the user's right wrist.

The sensor data D10BL includes velocity data and acceleration data of the user's wrist with respect to the x-axis, velocity data and acceleration data of the user's wrist on the y-axis, and velocity data and acceleration data of the user's wrist with respect to the z-axis in the local coordinate system. The controller 27 acquires the sensor data D10BL from the sensor device 10B.

<Case C6>

The controller 27 may select the case C6 if the sensor devices 10 that transmitted sensor data to the electronic device 20 include the sensor devices 10A and 10B and the sensor device 10E-1 or the sensor device 10E-2.

In the case C6, sensor data representing the movement of the user's head, sensor data representing the movement of either one of the user's two wrists, and sensor data representing the movement of either one of the user's two ankles are used. In the case C6, the sensor data D10AG, the sensor data D10AL, the sensor data D10BL, and the sensor data D10EL-1 or the sensor data D10EL-2 are used.

<Case C7>

The controller 27 may select the case C7 if the sensor devices 10 that transmitted sensor data to the electronic device 20 include the sensor devices 10A and 10B, and the sensor device 10F-1 or the sensor device 10F-2.
In the case C7, sensor data representing the movement of the user's head, sensor data representing the movement of either one of the user's two wrists, and sensor data representing the movement of either one of the user's two feet are used. In the case C7, the sensor data D10AG, the sensor data D10AL, the sensor data D10BL, and the sensor data D10FL-1 or the sensor data D10FL-2 are used.

<Case C8>

The controller 27 may select the case C8 if the sensor devices 10 that transmitted sensor data to the electronic device 20 include the sensor devices 10A, 10B, 10F-1, and 10F-2.
In the case C8, sensor data representing the movement of the user's head, sensor data representing the movement of either one of the user's two wrists, and sensor data representing the movement of each of the user's two feet are used. In the case C8, the sensor data D10AG, the sensor data D10AL, the sensor data D10BL, the sensor data D10FL-1, and the sensor data D10FL-2 are used.

<Case C9>

The controller 27 may select the case C9 if the sensor devices 10 that transmitted sensor data to the electronic device 20 include the sensor device 10F-1 and the sensor device 10F-2.
In the case C9, sensor data representing the movement of each of the user's two feet is used. In the case C9, the sensor data D10FL-1 and the sensor data D10FL-2 are used.

<Case C10>

The controller 27 may select the case C10 if the sensor devices 10 that transmitted sensor data to the electronic device 20 include the sensor devices 10D-1 and 10D-2.
In the case C10, sensor data representing the movement of each of the user's two thighs is used. In the case C10, the sensor data D10DL-1 and the sensor data D10DL-2 are used.
The sensor data D10DL-1 is sensor data representing the movement of the user's left thigh in the local coordinate system with respect to the position of the sensor device 10D-1. The sensor data D10DL-1 includes velocity data and acceleration data of the user's left thigh with respect to the x-axis, velocity data and acceleration data of the user's left thigh with respect to the y-axis, and velocity data and acceleration data of the user's left thigh with respect to the z-axis in the local coordinate system. The controller 27 acquires the sensor data D10DL-1 from the sensor device 10D-1.
The sensor data D10DL-2 is sensor data representing the movement of the user's right thigh in the local coordinate system with respect to the position of the sensor device 10D-2. The sensor data D10DL-2 includes velocity data and acceleration data of the user's right thigh with respect to the x-axis, velocity data and acceleration data of the user's right thigh with respect to the y-axis, and velocity data and acceleration data of the user's right thigh with respect to the z-axis in the local coordinate system. The controller 27 acquires the sensor data D10DL-2 from the sensor device 10D-2.

<Case C11>

The controller 27 may select the case C11 if the sensor devices 10 that transmitted sensor data to the electronic device 20 include the sensor device 10C.
In the case C11, sensor data representing the movement of the user's waist is used. In the case C11, sensor data D10CG and sensor data D10CL are used.
The sensor data D10CG is sensor data representing the movement of the user's waist in the global coordinate system. The sensor data D10CG includes velocity data and acceleration data of the user's waist with respect to the X-axis, velocity data and acceleration data of the user's waist with respect to the Y-axis, and velocity data and acceleration data of the user's waist with respect to the Z-axis in the global coordinate system. The controller 27 acquires the sensor data D10CG by performing a coordinate transformation on the sensor data in the local coordinate system acquired from the sensor device 10C.

The sensor data D10CL is sensor data representing the movement of the user's waist in the local coordinate system with respect to the position of the sensor device 10C. The sensor data D10CL includes velocity data and acceleration data of the user's waist with respect to the x-axis, velocity data and acceleration data of the user's waist with respect to the y-axis, and velocity data and acceleration data of the user's waist with respect to the z-axis in the local coordinate system. The controller 27 acquires the sensor data D10CL from the sensor device 10C.

<Case C12>

The controller 27 may select the case C12 if the sensor devices 10 that transmitted sensor data to the electronic device 20 include the sensor device 10B and the sensor device 10C.

In the case C12, sensor data representing the movement of either one of the user's two wrists and sensor data representing the movement of the user's waist are used. In the case C12, the sensor data D10BL, the sensor data D10CG, and the sensor data D10CL are used.

<Case C13>

The controller 27 may select the case C13 if the sensor devices 10 that transmitted sensor data to the electronic device 20 include the sensor devices 10B, 10F-1, 10F-2, and 10C.

In the case C13, sensor data representing the movement of either one of the user's two wrists, sensor data representing the movement of each of the user's two feet, and sensor data representing the movement of the user's waist are used. In the case C13, the sensor data D10BL, the sensor data D10FL-1, the sensor data D10FL-2, the sensor data D10CG, and the sensor data D10CL are used.

[Generation and Evaluation of Transformer]

Next, generation and evaluation of a transformer will be described. The inventors generated and evaluated a transformer that outputs estimated values of posture angles of body parts across the user's entire body and an estimated value of the normalized velocity of the user's waist. The user's body parts across the user's entire body include the user's head, neck, chest, lumbar spine, pelvis, right and left thighs, right and left lower legs, right and left feet, right and left upper arms, and right and left forearms.

A subject gait database was used in the generation of the transformer. As the subject gait database, data provided in "Y Kobayashi, N. Hida, K. Nakajima, M. Fujimoto, M. Mochimaru, "2019: AIST Gait Database 2019," [Online], [retrieved November 11, 2021], Internet <https ://unit.aist.go.jp/harc/ExPART/GDB2019_e.html>" was used. Gait data of multiple subjects are registered in this gait database. The gait data of a subject includes data representing the movement of the subject while walking and data on the ground reaction force applied to the subject while walking. The data representing the movement of a subject while walking was detected by a motion capture system. The data on the ground reaction force applied to the subject while walking was detected by a ground reaction force meter.

From the data representing the subject's movements detected by the motion capture system in the gait database described above, data corresponding to the sensor data, data of the posture angles of the subject's body parts, and data of the movement velocity of the waist were acquired. Data of the normalized velocity of the subject's waist was calculated by dividing the movement velocity of the subject's waist by the subject's height. Data sets were generated by mapping the data corresponding to the sensor data to the data of the posture angles of the body parts and the data of the normalized velocity of the waist. Data sets corresponding to the cases C1 to C13 described above with reference to FIG. 7 were generated. Sensor data representing the movement of the subject's left wrist was used as the sensor data D10BL for the case C6 described above with reference to FIG. 7. Out of the sensor data D10EL-1 and the sensor data D10EL-2, the sensor data D10EL-1 representing the movement of the user's left ankle was used in the case C2 and the case C6. Out of the sensor data D10FL-1 and the sensor data D10FL-2, the sensor data D10FL-1 representing the movement of the left foot was used in the case C3 and the case C7. Out of the sensor data D10DL-1 and the sensor data D10DL-2, the sensor data D10DL-1 representing the movement of the left thigh is used in the case C4.

Training of the transformer was performed with the generated datasets. In the training of the transformer, the datasets were given about 10% noise in order to counteract over-training.

The inventors evaluated the trained transformer by using data sets that were not used in training of the transformer. The inventors obtained evaluation results for the cases C1 to C13 described above with reference to FIG. 7.

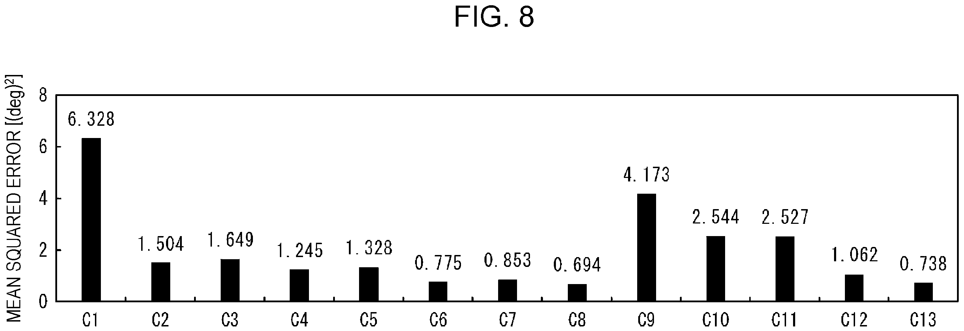

A graph of the evaluation results is illustrated in FIG. 8. FIG. 8 illustrates a bar chart of the Mean Squared Error (MSE) of the estimated values of the posture angles etc. of the body parts across the entire subject's body for each of the cases C1 to C13 as evaluation results. The mean squared error data depicted in FIG. 8 were obtained from the subjects illustrated in FIG. 9, described below. The mean squared errors were calculated based on the estimated values of posture angles and the movement velocity of the waist from the transformer and the measured values of posture angles and movement velocity of the waist from the data sets. The mean squared errors were calculated using Eq. (6) below.

[Math 6]

$$MSE_{seq} = \frac{1}{n} \times \frac{1}{d} \sum_{j=1}^{n} \sum_{i=1}^{d} \left(a_{i,j} - b_{i,j}\right)^2 \qquad \text{Eq. (6)}$$

In Eq. (6), j corresponds to the X-axis, the Y-axis, and the Z-axis of the global coordinate system. d is the number of dimensions of the global coordinate system, i.e., 3. $a_{i,j}$ is the measured value of the posture angle of a body part or the measured value of the movement velocity of the waist. $b_{i,j}$ is the estimated value of the posture angle of the body part when $a_{i,j}$ is the measured value of the posture angle of the body part, $b_{i,j}$ is the estimated value of the movement velocity of the waist when $a_{i,j}$ is the measured value of the movement velocity of the waist. N is the number of posture angle samples.

As illustrated in FIG. 8, in the case C1, the mean squared error was 6.328 $[(deg)^2]$. In the case C1, only sensor data representing the movement of the user's head is used. The results of the case C 1 demonstrate that even with only sensor data representing the movement of the user's head, the posture angles of body parts across the user's entire body can be estimated with some degree of accuracy. The reason for this is presumably that the movement of the user in the up-down direction while walking is reflected in the movement of the head.

As illustrated in FIG. 8, the mean squared errors in the cases C2 to C8 were smaller than the mean squared error in the case C 1. In other words, the estimation accuracy of the posture angles of the user's body parts in the cases C2 to C8 was improved compared to that in the case C1. In the cases C2 to C8, sensor data representing the movement of at least any of the user's wrists, ankles, feet, and thighs is used in addition to the sensor data representing the movement of the user's head, as described above. In other words, in the cases C2 to C8, sensor data representing the movement of the user's limbs, including at least any of the user's wrists, ankles, feet, and thighs, are used in addition to sensor data representing the movement of the user's torso including the user's head. Sensor data representing the movement of the user's limbs and sensor data representing the movement of the user's torso have significantly different patterns from each other within a single gait cycle. For example, due to the left-right symmetry of the user's body, the sensor data representing the movement of the user's limbs have a single pattern within a single gait cycle. In contrast, sensor data representing the movement of the user's torso have two patterns within a single gait cycle. The estimation accuracy of the posture angles of the body parts in the cases C2 to C8 being better than that in the case C1 is presumed to be because sensor data having different patterns within a single gait cycle are used.

As illustrated in FIG. 8, in the case C9, the mean squared error was 4.173 $[(deg)^2]$. In the case C10, the mean squared error was 2.544 $[(deg)^2]$. In the cases C9 and C10, sensor data representing the movement of the user's feet and sensor data representing the movement of the user's thighs are respectively used. The feet and thighs are body parts that are highly relevant to walking among the user's body parts. In the cases C9 and C10, the posture angles of the body parts are presumed to be able to be estimated with some degree of accuracy because the sensor data representing the movement of feet or thighs, which are body parts that are highly relevant to walking, are used.

As illustrated in FIG. 8, in the case C11, the mean squared error was 2.527 $[(deg)^2]$. In the case C 11, only sensor data representing the movement of the user's waist is used. The results of the case C 11 demonstrate that even with only sensor data representing the movement of the user's waist, the posture angles of the user's body parts can be estimated with some degree of accuracy. The reason for this is presumably that the movement of the user in the up-down direction while walking is reflected in the movement of the torso including the waist.

As illustrated in FIG. 8, the mean squared errors in the cases C12 and C13 were smaller than the mean squared error in the case C11. In other words, the estimation accuracy of the posture angles of the user's body parts in the cases C12 and C13 was improved compared to that in the case C11. In the cases C12 and C13, sensor data representing the movement of at least any of the user's wrists and ankles is used in addition to the sensor data representing the movement of the user's waist, as described above. In other words, in the cases C12 and C13, sensor data representing the movement of the user's limbs, including at least any of the user's wrists and ankles, are used in addition to sensor data representing the movement of the user's torso including the user's waist. As described above, sensor data representing the movement of the user's limbs and sensor data representing the movement of the user's torso have significantly different patterns from each other within a single gait cycle. The estimation accuracy of the posture angles of the body parts in the cases C12 and C13 being better than that in the case C11 is presumed to be because sensor data having different patterns within a single gait cycle are used.

As illustrated in FIG. 8, in each of C06, C07, C08, and C13, the mean squared error was less than 1.0 $[(deg)^2]$. The estimation accuracy of the posture angles was high in C06, C07, C08, and C13 among the cases C1 to C13.

Next, comparison results comparing measured values and estimated values of the posture angles of body parts of subjects will be described. First, the subjects used in the comparison results will be described while referring to FIG. 9.

FIG. 9 illustrates an example of subjects. The subjects have diverse physical characteristics.

A subject SU1 is male, 33 years of age, has a height of 171 [cm], and a weight of 100 [kg]. Physical characteristics of the subject SU1 are that he is a heavy weight male.

A subject SU2 is female, 70 years of age, has a height of 151 [cm], and a weight of 39 [kg]. Physical characteristics of the subject SU2 are that she is a light weight female.

A subject SU3 is female, 38 years of age, has a height of 155 [cm], and a weight of 41 [kg]. Physical characteristics of the subject SU3 are that she is a light weight young female.

A subject SU4 is female, 65 years of age, has a height of 149 [cm], and a weight of 70 [kg]. Physical characteristics of the subject SU4 are that she is a heavy weight female.

A subject SU5 is male, 22 years of age, has a height of 163 [cm], and a weight of 65 [kg]. Physical characteristics of subject SU5 are that he is a male of average height and average weight.

A subject SU6 is female, 66 years of age, has a height of 149 [cm], and a weight of 47 [kg]. Physical characteristics of the subject SU6 are that she is a short female.

A subject SU7 is female, 65 years of age, has a height of 148 [cm], and a weight of 47 [kg]. Physical characteristic of the subject SU7 are that she is a short female.

A subject SU8 is male, 57 years of age, has a height of 178 [cm], and a weight of 81 [kg]. Physical characteristics of the subject SU8 are that he is a tall male.

<Comparison Results 1>

As Comparison Results 1, the measured values and estimated values of the posture angles of different body parts of one subject SU6 obtained when using the sensor data in the case C6 are described.

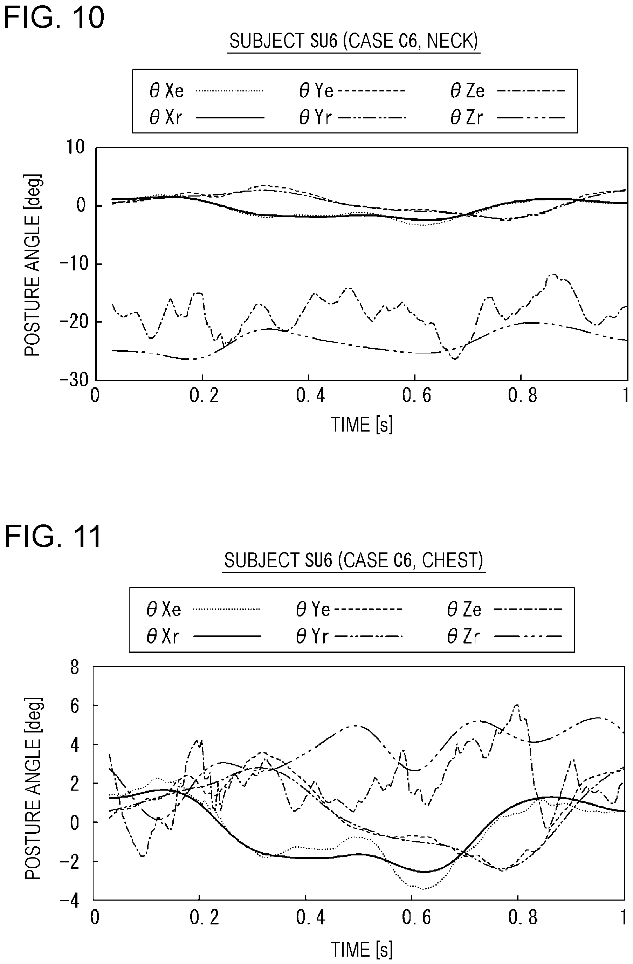

FIGs. 10 to 21 illustrate graphs of the measured values and estimated values of the posture angles of the body parts of the subject SU6. The horizontal axis in FIGs. 10 to 21 represents time [s]. The vertical axis in FIGs. 10 to 21 represents posture angle [deg].

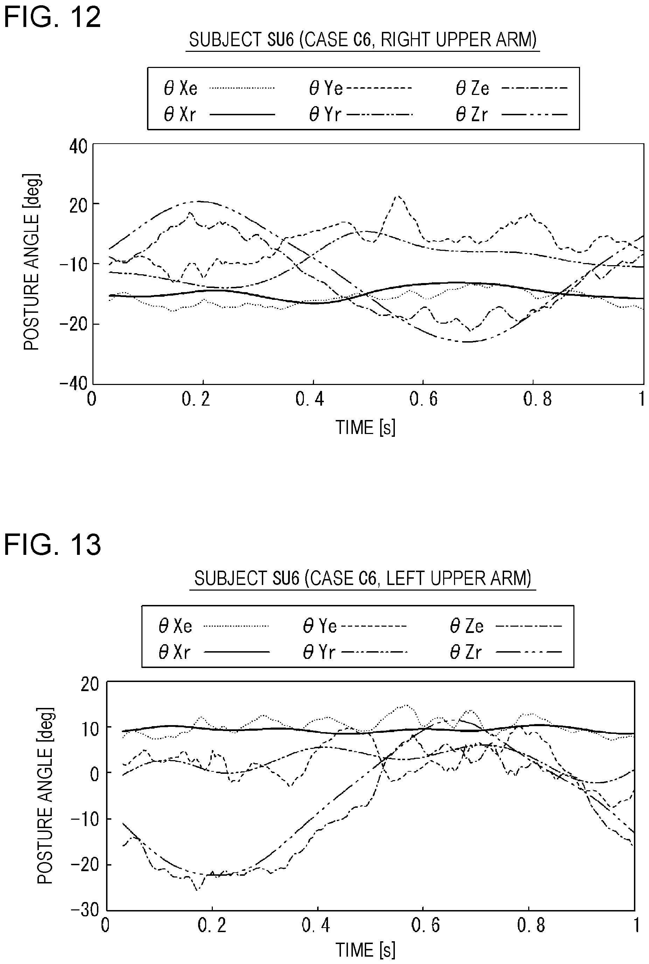

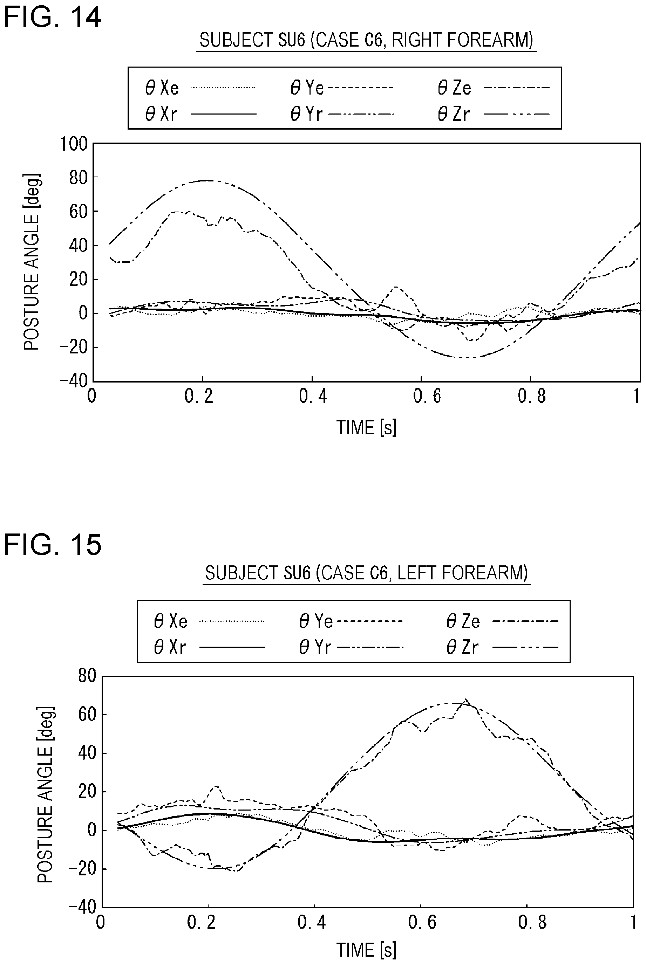

FIGs. 10 to 15 are graphs of the posture angles of the body parts of the upper body of the subject SU6. Specifically, FIG. 10 is a graph of the posture angle of the neck of the subject SU6. FIG. 11 is a graph of the posture angle of the chest of the subject SU6. FIG. 12 is a graph of the posture angle of the right upper arm of the subject SU6. FIG. 13 is a graph of the posture angle of the left upper arm of the subject SU6. FIG. 14 is a graph of the posture angle of the right forearm of the subject SU6. FIG. 15 is a graph of the posture angle of the left forearm of the subject SU6.

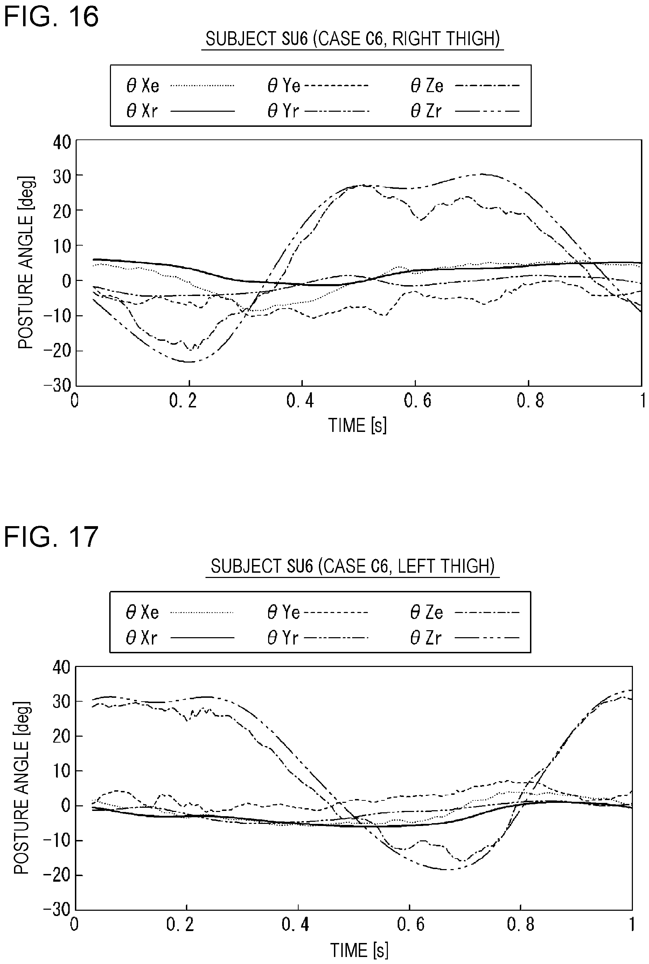

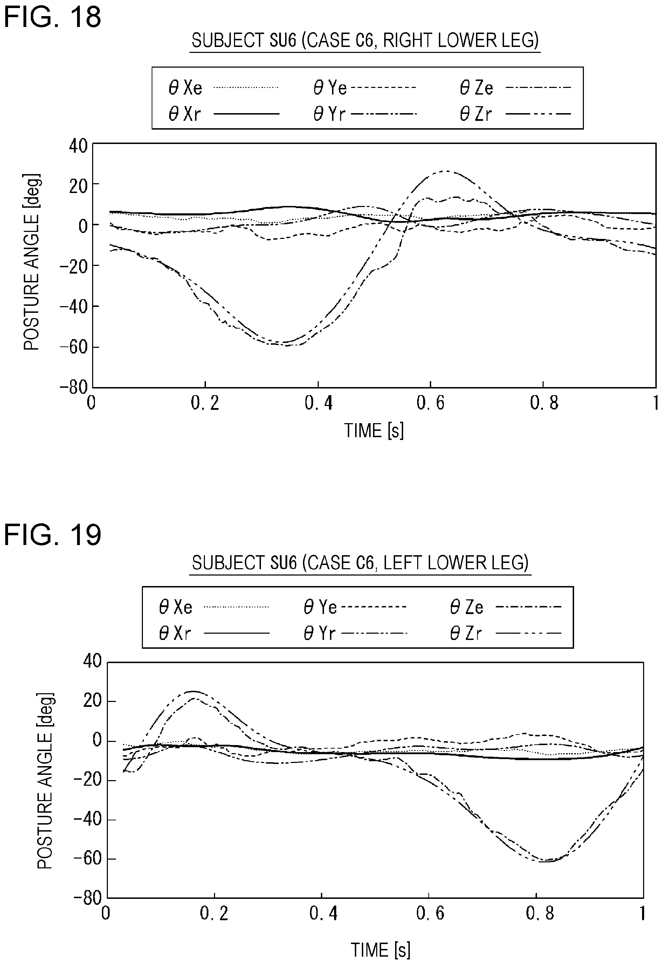

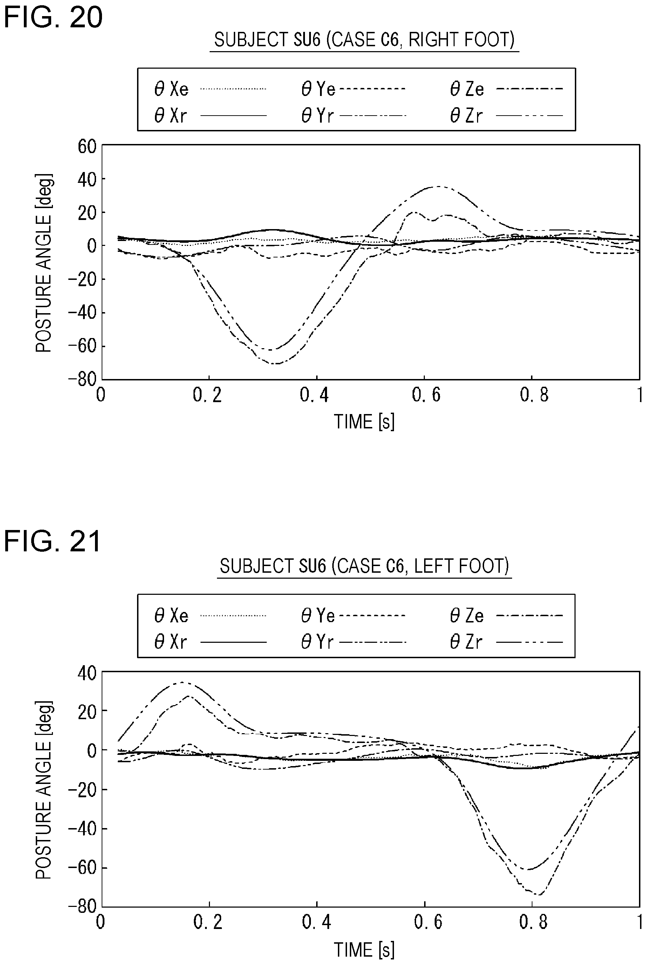

FIGs. 16 to 21 are graphs of the posture angles of the body parts of the lower body of the subject SU6. Specifically, FIG. 16 is a graph of the posture angle of the right thigh of the subject SU6. FIG. 17 is a graph of the posture angle of the left thigh of the subject SU6. FIG. 18 is a graph of the posture angle of the right lower leg of the subject SU6. FIG. 19 is a graph of the posture angle of the left lower leg of the subject SU6. FIG. 20 is a graph of the posture angle of the right foot of the subject SU6. FIG. 21 is a graph of the posture angle of the left foot of the subject SU6.

In the drawings below, a posture angle $\theta Xr$ is the measured value of the posture angle $\theta X$ described above. A posture angle $\theta Yr$ is the measured value of the posture angle $\theta Y$ described above. A posture angle $\theta Zr$ is the measured value of the posture angle $\theta Z$ described above.

In the drawings below, a posture angle $\theta Xe$ is the estimated value of the posture angle $\theta X$ described above. A posture angle $\theta Ye$ is the estimated value of the posture angle $\theta Y$ described above. A posture angle $\theta Ze$ is the estimated value of the posture angle $\theta Z$ described above.

As illustrated in FIGs. 10 to 15, the estimated values of the posture angles of the body parts of the upper body of the subject SU6 agreed relatively well with the measured values. As described above, sensor data representing the movement of the subject's left wrist was used as the sensor data D10BL, as illustrated in FIG. 7, for the case C6. In other words, in the case C6, sensor data representing the movement of the subject's right wrist is not used. Nevertheless, as illustrated in FIG. 12, the estimated values of the posture angle of the right upper arm of the subject SU6 matched the measured values of the posture angle of the right upper arm of the subject SU6 with the same or a similar degree of accuracy as the values for the left upper arm illustrated in FIG. 13. In addition, as illustrated in FIG. 14, the estimated values of the posture angle of the right forearm of the subject SU6 matched the measured values of the posture angle of the right forearm of the subject SU6 with the same or a similar degree of accuracy as the values for the left forearm illustrated in FIG. 15.

As illustrated in FIGs. 16 to 21, the estimated values of the posture angles of the body parts of the lower body of the subject SU6 agreed relatively well with the measured values. As described above, in the case C6, sensor data representing the movement of the subject's right wrist is not used. Nevertheless, as illustrated in FIG. 16, the estimated values of the posture angle of the right thigh of the subject SU6 matched the measured values of the posture angle of the right thigh of the subject SU6 with the same or a similar degree of accuracy as the values for the left thigh illustrated in FIG. 17. In addition, as illustrated in FIG. 18, the estimated values of the posture angle of the right lower leg of the subject SU6 matched the measured values of the posture angle of the right lower leg of the subject SU6 with the same or a similar degree of accuracy as the values for the left lower leg illustrated in FIG. 19. As illustrated in FIG. 20, the estimated values of the posture angle of the right foot of the subject SU6 matched the measured values of the posture angle of the right foot of the subject SU6 with the same or a similar degree of accuracy as the values for the left foot illustrated in FIG. 21.

<Comparison Results 2>

As Comparison Results 2, the measured values and estimated values of the posture angles of the right thighs of subjects evaluated as having a large center of gravity shift and subjects evaluated as having a small center of gravity shift for when the case C6 is used are described. A subject evaluated as having a large center of gravity shift means a subject for which the shift of the center of gravity of the subject in the up-down direction while walking is large. A subject evaluated as having a small center of gravity shift means a subject for which the shift of the center of gravity of the subject in the up-down direction while walking is small.

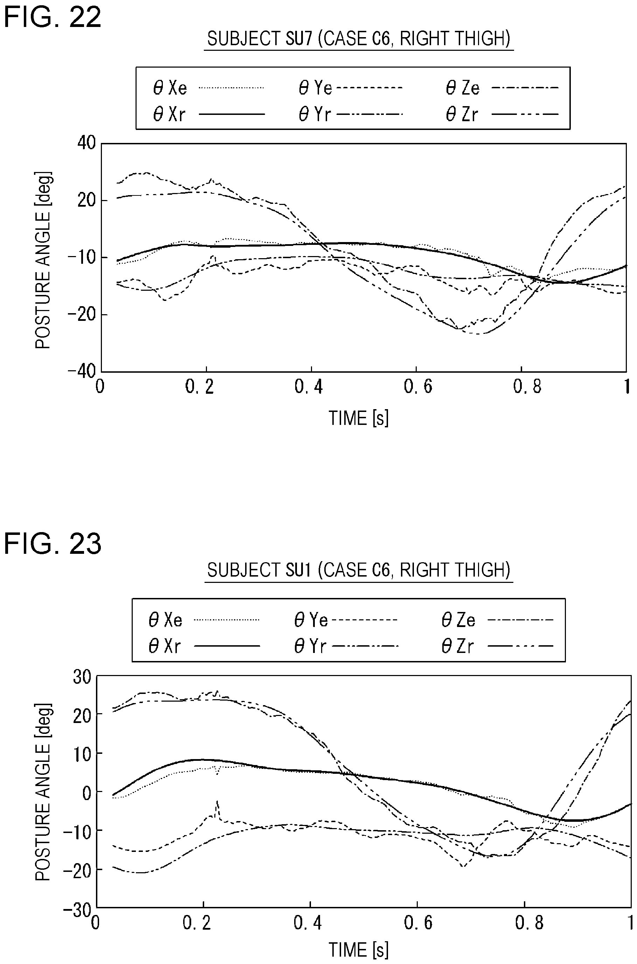

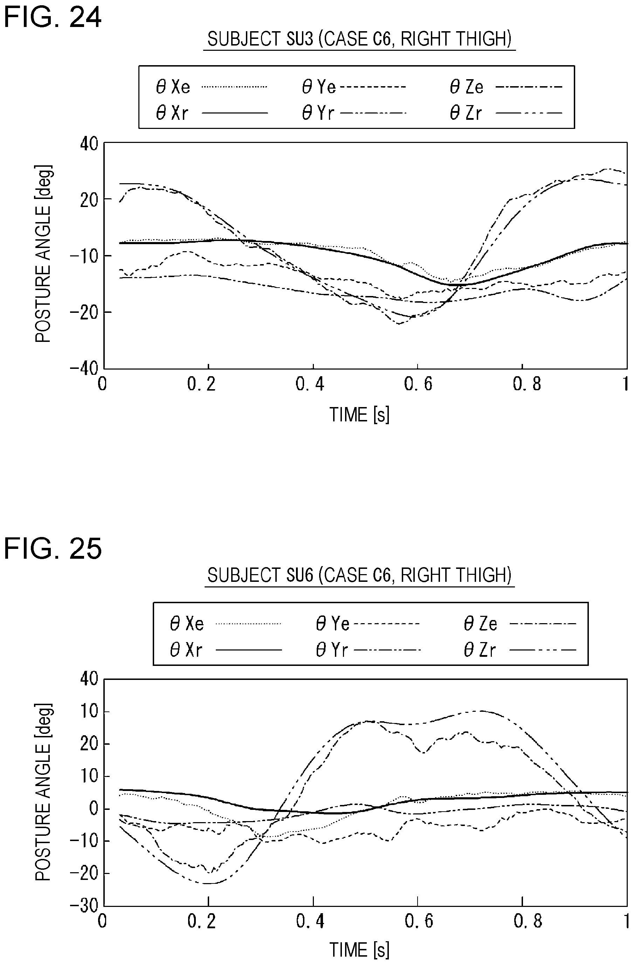

FIG. 22 is a graph of the posture angle of the right thigh of the subject SU7 evaluated as having a large center of gravity shift. FIG. 23 is a graph of the posture angle of the right thigh of the subject SU1 evaluated as having a large center of gravity shift. FIG. 24 is a graph of the posture angle of the right thigh of the subject SU3 evaluated as having a large center of gravity shift. FIG. 25 is a graph of the posture angle of the right thigh of the subject SU6 evaluated as having a large center of gravity shift.

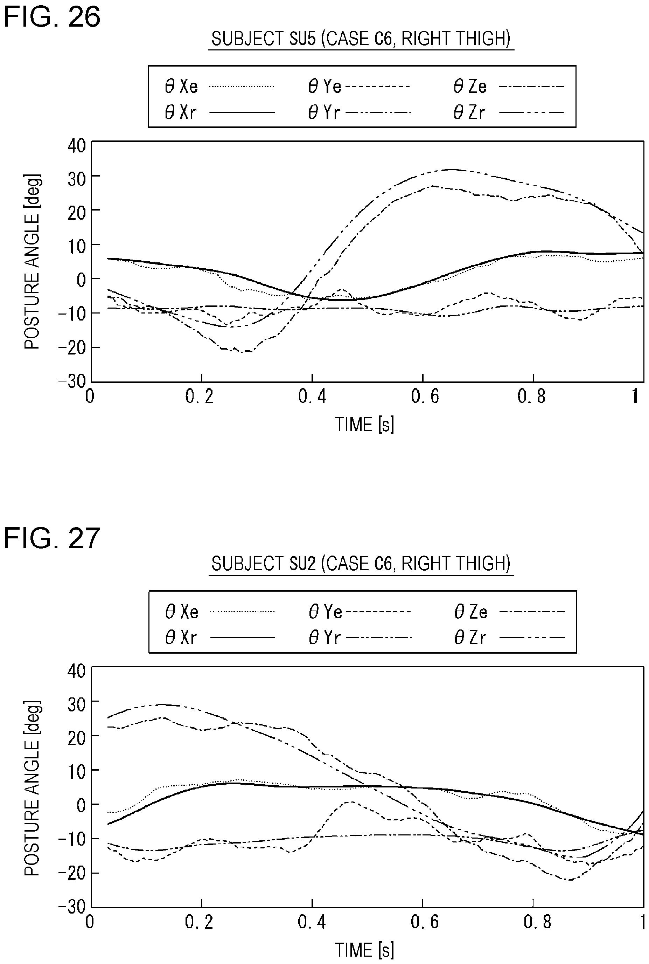

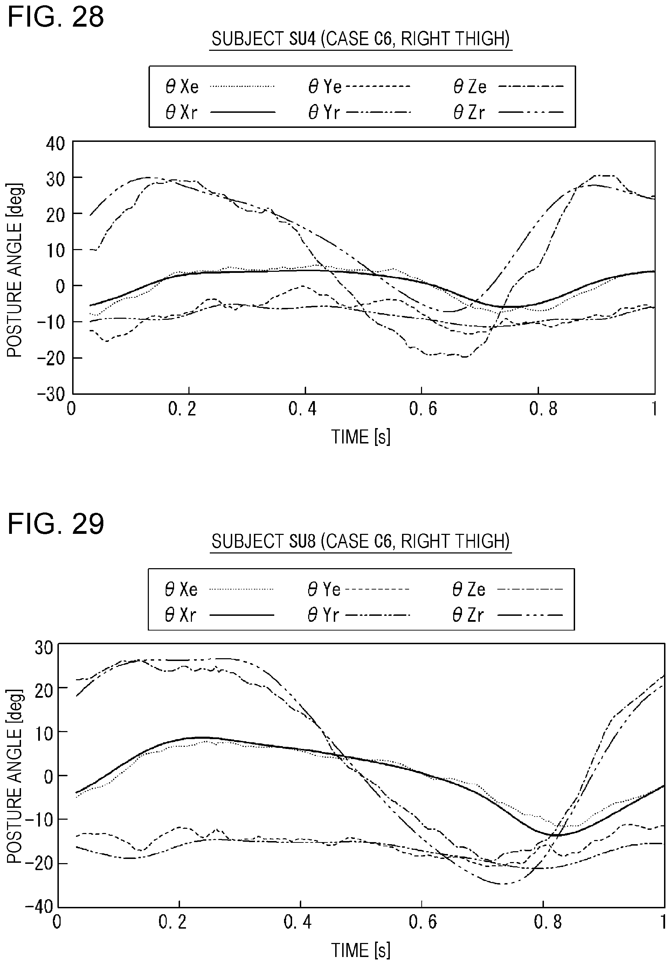

FIG. 26 is a graph of the posture angle of the right thigh of the subject SU5 evaluated as having a small center of gravity shift. FIG. 27 is a graph of the posture angle of the right thigh of the subject SU2 evaluated as having a small center of gravity shift. FIG. 28 is a graph of the posture angle of the right thigh of the subject SU4 evaluated as having a small center of gravity shift. FIG. 29 is a graph of the posture angle of the right thigh of the subject SU8 evaluated as having a small center of gravity shift.

The horizontal axis in FIGs. 22 to 29 represents time [s]. The vertical axis in FIGs. 22 to 29 represents posture angle [deg].

As described above, in the case C6, sensor data representing the movement of the subject's right wrist is not used. Nevertheless, as illustrated in FIGs. 22 to 25, the estimated values of the posture angle of the right thigh agreed relatively well with the measured values of the posture angle of the right thigh for the subjects evaluated as having a large center of gravity shift.

As described above, in the case C6, sensor data representing the movement of the subject's right wrist is not used. Nevertheless, as illustrated in FIGs. 26 to 29, the estimated values of the posture angle of the right thigh agreed relatively well with the measured values of the posture angle of the right thigh for the subjects evaluated as having a small center of gravity shift.

For the subjects evaluated as having a small center of gravity shift, the shift of the center of gravity of the subject in the up-down direction is smaller and the amount of movement of the subject in the up-down direction is also smaller than for the subjects evaluated as having a large center of gravity shift. Therefore, for the subjects evaluated as having a small center of gravity shift, the sensor data is less likely to reflect the movement of the subject in the up-down direction while walking than for the subjects evaluated as having a large center of gravity shift. Nevertheless, as illustrated in FIGs. 22 to 29, the estimated values of the posture angle of the right thigh of the subjects evaluated as having a small center of gravity shift matched the measured values with the same or a similar degree of accuracy as the values for the subjects evaluated as having a large center of gravity shift. This indicates that if the case C6 is selected, even for the subjects evaluated as having a small center of gravity shift, the posture angle of the right thigh can be estimated with the same or a similar degree of accuracy as for the subjects evaluated as having a large center of gravity shift.

<Comparison Results 3>

As Comparison Results 3, the measured values and estimated values of the posture angles of the right upper arms of subjects evaluated as having a large center of gravity shift and subjects evaluated as having a small center of gravity shift for when the case C6 is used are described.

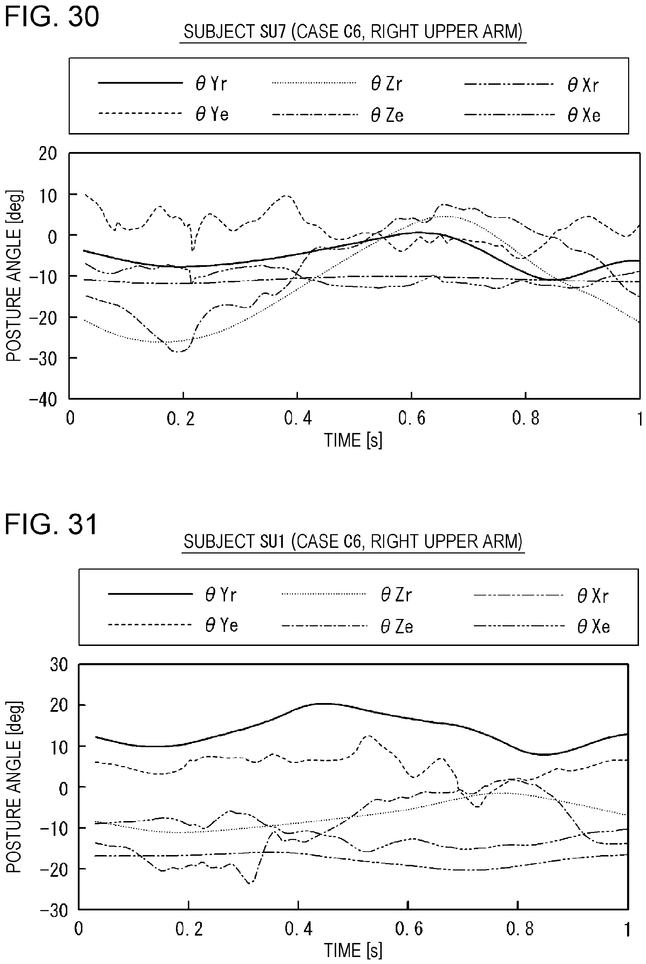

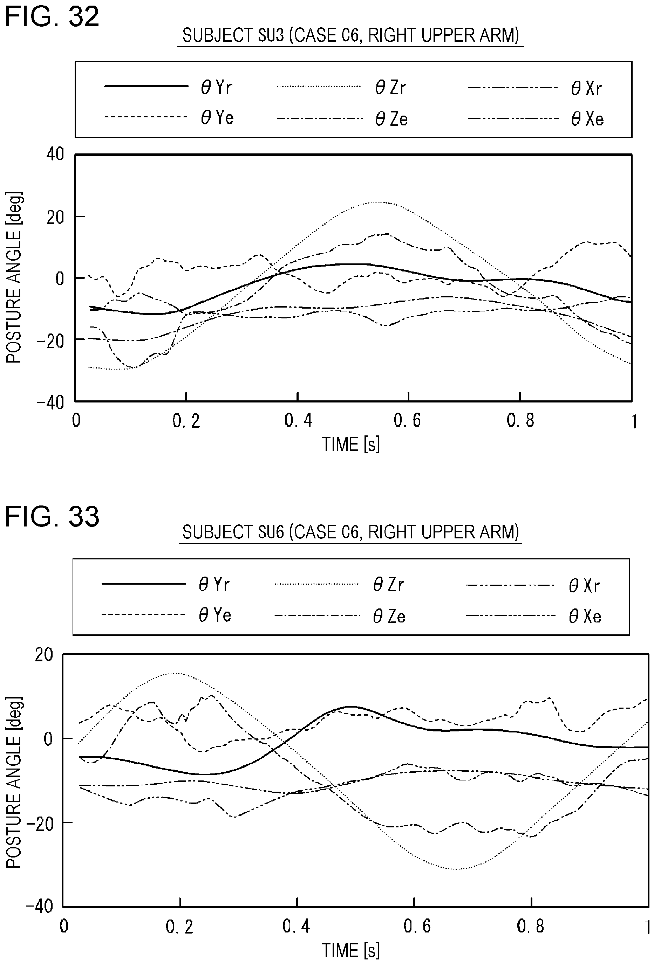

FIG. 30 is a graph of the posture angle of the right upper arm of the subject SU7 evaluated as having a large center of gravity shift. FIG. 31 is a graph of the posture angle of the right upper arm of the subject SU1 evaluated as having a large center of gravity shift. FIG. 32 is a graph of the posture angle of the right upper arm of the subject SU3 evaluated as having a large center of gravity shift. FIG. 33 is a graph of the posture angle of the right upper arm of the subject SU6 evaluated as having a large center of gravity shift.

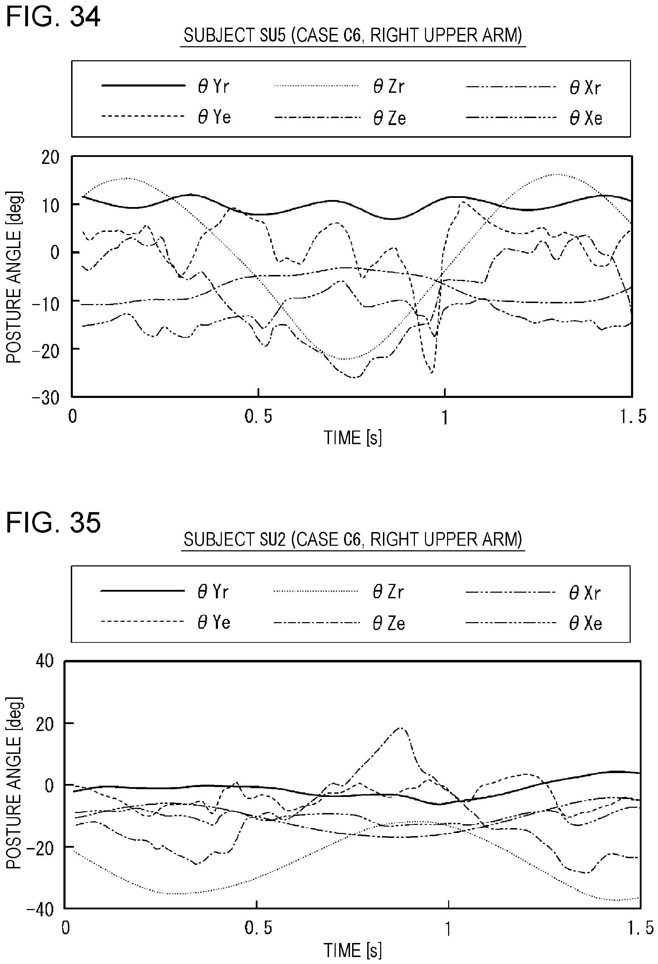

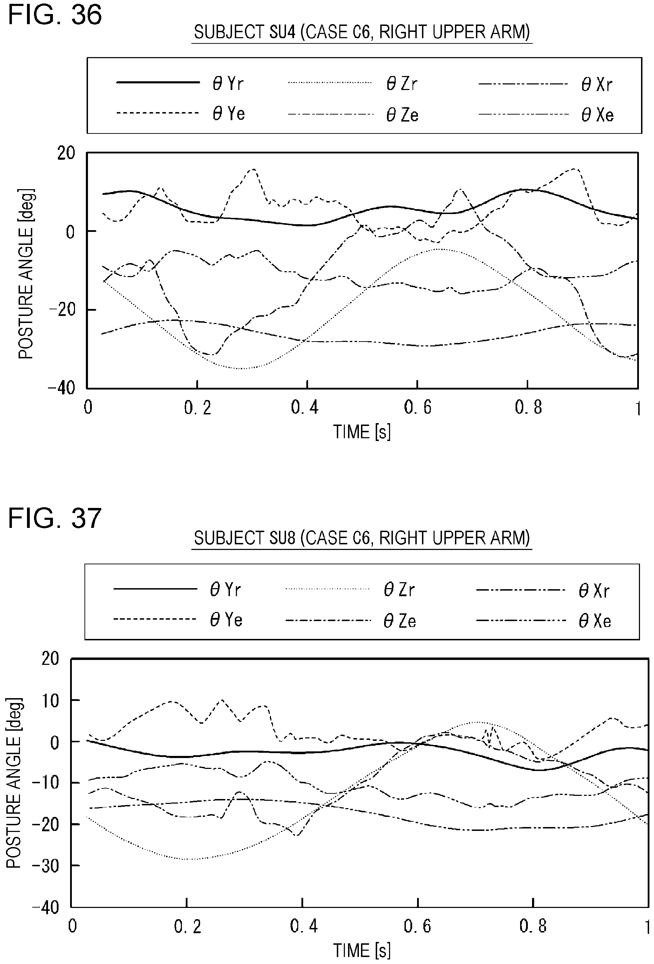

FIG. 34 is a graph of the posture angle of the right upper arm of the subject SU5 evaluated as having a small center of gravity shift. FIG. 35 is a graph of the posture angle of the right upper arm of the subject SU2 evaluated as having a small center of gravity shift. FIG. 36 is a graph of the posture angle of the right upper arm of the subject SU4 evaluated as having a small center of gravity shift. FIG. 37 is a graph of the posture angle of the right upper arm of the subject SU8 evaluated as having a small center of gravity shift.

The horizontal axis in FIGs. 30 to 37 represents time [s]. The vertical axis in FIGs. 30 to 37 represents posture angle [deg].

As described above, in the case C6, sensor data representing the movement of the subject's right wrist is not used. Nevertheless, as illustrated in FIGs. 30 to 37, the times at which the maxima and minima appeared in the estimated values of the posture angles of the right upper arms, etc., agreed relatively well with the measured values.

As described above, for subjects evaluated as having a small center of gravity shift, the amount of movement of the subject in the up-down direction is smaller, and therefore the sensor data is less likely to reflect the movement of the subject in the up-down direction while walking than for subjects evaluated as having a large center of gravity shift. Nevertheless, as illustrated in FIGs. 30 to 37, the estimated values of the posture angle of the right upper arm of the subjects evaluated as having a small center of gravity shift matched the measured values with the same or a similar degree of accuracy as for the subjects evaluated as having a large center of gravity shift. This indicates that if the case C6 is selected, even for the subjects evaluated as having a small center of gravity shift, the posture angle of the right upper arm can be estimated with the same or a similar degree of accuracy as for the subjects evaluated as having a large center of gravity shift.

[Evaluation Determination Processing]

The controller 27 may evaluate the user's gait based on estimated values of the posture angles of the user's body parts. As an example, the controller 27 may estimate the size of the shift of the user's center of gravity in the up-down direction based on the estimated values of the posture angles of the user's body parts and evaluate the user's center of gravity shift.

The controller 27 may inform the user of the determined evaluation using the notification unit 23. For example, the controller 27 may display information on the determined evaluation on the display of the output unit 24, or may output information on the determined evaluation as audio to the speaker of the output unit 24. Alternatively, the controller 27 may cause the vibration unit 25 to vibrate with a vibration pattern in accordance with the determined evaluation.

The controller 27 may generate an evaluation signal representing the determined evaluation. The controller 27 may transmit the generated evaluation signal to the communication unit 21 to any external device. As an example, the controller 27 may transmit the evaluation signal to any sensor device 10 including the notification unit 13 as an external device by using the communication unit 21. In this case, in the sensor device 10, the controller 16 receives the evaluation signal via the communication unit 11. The controller 16 causes the notification unit 13 to report the information represented by the evaluation signal. For example, the controller 16 causes the output unit 14 to output the information represented by the evaluation signal. This configuration allows the user to grasp the evaluation of his or her own gait.

The controller 27 may transmit the evaluation signal using the communication unit 21 to an earphone as an external device, if the sensor device 10A is an earphone or is contained in an earphone, for example. For example, in the sensor device 10A, the controller 16 causes the speaker of the output unit 14 to output the information represented by the evaluation signal as audio upon receiving the evaluation signal via the communication unit 11. This configuration allows the user to be informed of information on the evaluation of his or her gait via audio. Informing the user via audio reduces the likelihood of the user's walk being disturbed.

(System Operation)

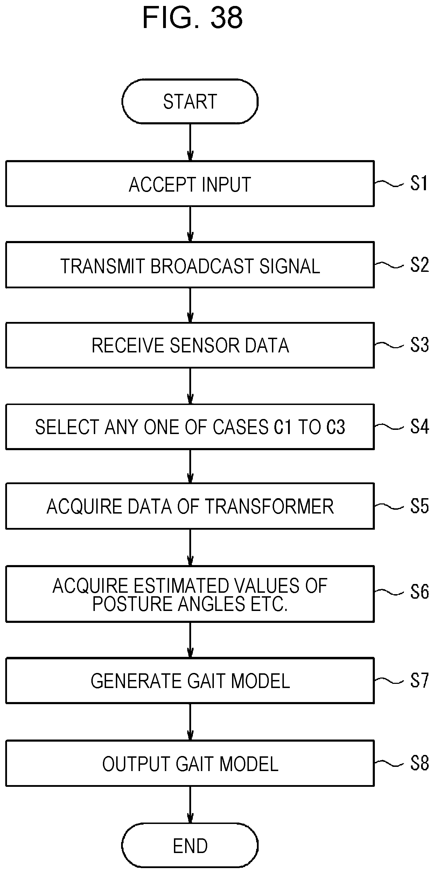

FIG. 38 is a flowchart illustrating operation of posture angle estimation processing performed by the electronic device 20 illustrated in FIG. 1. This operation corresponds to an example of an information processing method according to this embodiment. For example, when the user inputs an input instructing execution of the posture angle estimation processing from the input unit 22, the controller 27 starts the processing of Step S1.

The controller 27 accepts an input instructing execution of posture angle estimation processing via the input unit 22 (Step S1).

The controller 27 transmits a signal instructing the start of data detection to multiple sensor devices 10 as a broadcast signal via the communication unit 21 (Step S2). After the processing of Step S2 is performed, sensor data is transmitted from at least one sensor device 10 to the electronic device 20.

The controller 27 receives the sensor data from the at least one sensor device 10 via the communication unit 21 (Step S3).

The controller 27 selects any of the cases C1 to C13 in accordance with the type of sensor device 10 that transmitted the sensor data to the electronic device 20 (Step S4). The controller 27 acquires the data of the transformer 30 used in the cases C1 to C13 selected in the processing of Step S4 from the storage unit 26 (Step S5).

The controller 27 inputs the sensor data for the cases C1 to C13 selected in the processing of Step S4 to the transformer whose data was acquired in the processing of Step S5. The controller 27 inputs the sensor data to the transformer and acquires from the transformer time-series data of the estimated values of the posture angles of the body parts across the user's entire body and time-series data of the movement velocity of the user's waist (Step S6).

The controller 27 generates a gait model based on the time-series data of the estimated values of the posture angles of the body parts across the user's entire body and the time-series data of the movement velocity of the user's waist acquired in the processing of Step S6 (Step S7). The controller 27 causes the output unit 24 to output data of the gait model generated in the processing of Step S7 (Step S8).

After executing the processing of Step S8, the controller 27 terminates the estimation processing. The controller 27 may perform the estimation processing again after terminating the estimation processing. In the estimation processing to be performed again, the controller 27 may start from the processing of Step S3. The controller 27 may repeat the estimation processing until an input instructing termination of the estimation processing is received from the input unit 22. An input instructing termination of the estimation processing is input by the user, for example, through the input unit 22. When the user finishes walking, for example, he or she inputs an input from the input unit 22 instructing termination of the estimation processing. When the controller 27 receives an input instructing termination of the estimation processing, the controller 27 may transmit a signal instructing termination of data detection as a broadcast signal to the multiple sensor devices 10 via the communication unit 21. In the sensor device 10, the controller 16 may terminate data detection when a signal instructing termination of data detection is received by the communication unit 11.

Thus, in the electronic device 20 serving as an information processing device, the controller 27 acquires estimated values of the posture angles of the user's body parts by using sensor data and a trained model. The user's movements can be detected by acquiring the estimated values of the posture angles of the user's body parts. In addition, the results illustrated in FIGs. 8 to 37 demonstrate that the sensor data representing the movement of one body part, out of parts of the user's body located on the left and right sides, and the trained model can be used to obtain estimated values of the posture angle of that body part on the other side of the user's body.

As a comparative example, let us consider a case where a camera is used to capture footage of the way in which the user walks and the data captured by the camera is analyzed in order to detect the user's movement. In this case, installation of the camera is required. Installation of the camera requires time and effort. In addition, the camera can only capture the way in which the user walks at the location where the camera is installed. Furthermore, depending on the clothing worn by the user, the movement of the user's body may not be visible from the outside due to the clothing. If the movement of the user's body are not visible from the outside, the camera might not be able to capture the movement of the user's body.

In contrast to this comparative example, this embodiment does not require the installation of a camera, and therefore the movement of the user can be detected more conveniently. In addition, regardless of where the user walks, so long as the user is wearing the sensor device 10, sensor data can be detected, and therefore the user's movement can be detected. Even if the movement of the user's body is not visible from the outside due to clothing, so long as the user is wearing the sensor device 10, the sensor data can be detected, and therefore the user's movement can be detected. So long as the user is wearing the sensor device 10, the user's movement can be detected regardless of where the user walks and for how long the user walks.

Therefore, according to this embodiment, an improved technology for detecting user movement can be provided.

Furthermore, in this embodiment, the transformer may be trained to output estimated values of the posture angles of the user's body parts when input with the sensor data for the case C1. The sensor data for the case C1 is detected by the sensor device 10A. With this configuration, estimated values of the body parts of the user can be acquired even when the user wears only the sensor device 10A. In addition, user convenience can be improved because the user only needs to wear the sensor device 10A. Furthermore, if the sensor device 10A is an earphone or is included in an earphone, the user can easily wear sensor device 10A on his or her head. The user being able to easily wear the sensor device 10A on his or her head further improves user convenience. In addition, when only the sensor data detected by sensor device 10A is used, the timings at which multiple sensor devices 10 detect data no longer need to be synchronized. By eliminating the need to synchronize the timings at which the multiple sensor devices 10 detect data, the estimated values of the posture angles can be acquired more easily.

Furthermore, in this embodiment, the transformer may be trained to output estimated values of the posture angles of the user's body parts when input with the sensor data for any one of the cases C2 to C5. The sensor data for the case C2 is detected by the sensor device 10A and the sensor device 10E-1 or 10E-2, i.e., by two sensor devices 10. The sensor data in the case C3 is detected by the sensor device 10A and the sensor device 10F-1 or 10F-2, i.e., by two sensor devices 10. The sensor data in the case C4 is detected by the sensor device 10A and the sensor device 10D-1 or 10D-2, i.e., by two sensor devices 10. The sensor data in the case C5 is detected by the sensor device 10A and by the sensor device 10B, i.e., by two sensor devices 10. Thus, since the sensor data is detected by two sensor devices 10 in the cases C2 to C5, the user only needs to wear two sensor devices 10. Therefore, user convenience can be improved. In addition, as described above with reference to FIG. 8, the estimation accuracy of the posture angles of the user's body parts was improved in the cases C2 to C5 compared to the case C1. Therefore, the posture angles of the user's body parts can be estimated with good accuracy by using the sensor data in the cases C2 to C5.

Furthermore, in this embodiment, the transformer may be trained to output estimated values of the posture angles of the user's body parts when input with the sensor data for the case C6 or C7. The sensor data in the case C6 is detected by the sensor device 10A, the sensor device 10B, and the sensor device 10E-1 or 10E-2, i.e., by three sensor devices 10. The sensor data in the case C7 is detected by the sensor device 10A, the sensor device 10B, and the sensor device 10F-1 or 10F-2, i.e., by three sensor devices 10. Thus, since the sensor data is detected by three sensor devices 10 in the cases C6 and C7, the user only needs to wear three sensor devices 10. Therefore, user convenience can be improved. In addition, as described above with reference to FIG. 8, the estimation accuracy of the posture angles of the user's body parts in the cases C6 and C7 was improved compared to that in the case C1. Therefore, the posture angles of the user's body parts can be estimated with good accuracy by using the sensor data in the cases C6 and C7.

(Another System Configuration)

FIG. 39 is a functional block diagram illustrating the configuration of an information processing system 101 according to another embodiment of the present disclosure.

The information processing system 101 includes the sensor device 10, the electronic device 20, and a server 80. In the information processing system 101, the server 80 functions as an information processing device and acquires estimated values of the posture angles of the user's body parts by using sensor data detected by the sensor device 10 and a trained model.

The electronic device 20 and the server 80 can communicate with each other via the network 2. The network 2 may be any network, including mobile object communication networks and the Internet.

The controller 27 of the electronic device 20 receives sensor data from the sensor device 10 via the communication unit 21, in the same or a similar manner to information processing system 1. In the information processing system 101, the controller 27 transmits sensor data to the server 80 via the network 2 by using the communication unit 21.

The server 80 is a server belonging to, for example, a cloud computing system or another computing system. The server 80 includes a communication unit 81, a storage unit 82, and a controller 83.

The communication unit 81 includes at least one communication module that can connect to the network 2. The communication module is, for example, a communication module that is compatible with standards such as wired LAN (Local Area Network) or wireless LAN. The communication unit 81 is connected to the network 2 via wired LAN or wireless LAN using the communication module.

The storage unit 82 includes at least one semiconductor memory, at least one magnetic memory, at least one optical memory, or a combination of at least two of these types of memories. The semiconductor memory is, for example, a RAM or a ROM. The RAM is, for example, an SRAM or a DRAM. The ROM is, for example, an EEPROM. The storage unit 82 may function as a main storage device, an auxiliary storage device, or a cache memory. The storage unit 82 stores data used in operation of the server 80 and data obtained through operation of the server 80. For example, the storage unit 82 stores system programs, application programs, and embedded software. For example, the storage unit 82 stores data of the transformer 30 as illustrated in FIG. 4 and data used by the transformer 30.

The controller 83 includes at least one processor, at least one dedicated circuit, or a combination thereof. The processor can be a general-purpose processor such as a CPU or a GPU, or a dedicated processor specialized for particular processing. The dedicated circuit is, for example, an FPGA or an ASIC. The controller 83 executes processing relating to operation of the server 80 while controlling the various parts of the server 80. The controller 83 may perform the processing to be performed by the transformer 30 as illustrated in FIG. 4.

The controller 83 uses the communication unit 81 to receive sensor data from the electronic device 20 via the network 2. The controller 83 acquires estimated values of the posture angles of the user's body parts based on sensor data and a trained model by executing processing the same as or similar to the processing executed by the controller 27 of the electronic device 20 described above.

(Other System Operations)

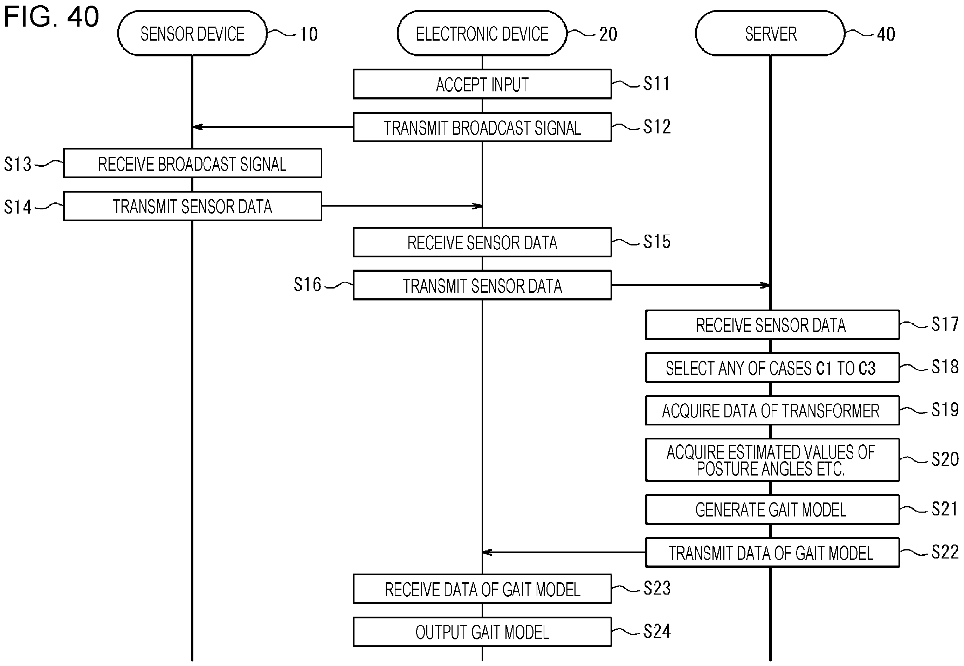

FIG. 40 is a sequence diagram illustrating operation of estimation processing performed by the information processing system 101 illustrated in FIG. 39. This operation corresponds to an example of an information processing method according to this embodiment. For example, when the user inputs an input instructing execution of the posture angle estimation processing from the input unit 22 of the electronic device 20, the information processing system 101 starts the processing of Step S1.

In the electronic device 20, the controller 27 accepts an input instructing execution of posture angle estimation processing via the input unit 22 (Step S11). The controller 27 transmits a signal instructing the start of data detection to the multiple sensor devices 10 as a broadcast signal via the communication unit 21 (Step S12).

In each sensor device 10, the controller 16 receives a signal instructing the start of data detection from the electronic device 20 via the communication unit 11 (Step S13). Upon receiving this signal, the controller 16 starts data detection. The controller 16 acquires data detected by the sensor unit 12 from the sensor unit 12. The controller 16 transmits the acquired data, as sensor data, to the electronic device 20 via the communication unit 11 (Step S14).

In the electronic device 20, the controller 27 receives sensor data from the sensor device 10 via the communication unit 21 (Step S15). The controller 27 transmits the sensor data to the server 80 via the network 2 by using the communication unit 21 (Step S16).

In the server 80, the controller 83 receives the sensor data from the electronic device 20 via the network 2 by using the communication unit 81 (Step S17). The controller 83 selects any one of the cases C1 to C13 in accordance with the type of sensor device 10 that transmitted the sensor data to the server 80 via the electronic device 20 (Step S18). The controller 83 acquires the data of the transformer 30 used in the cases C1 to C13 selected in the processing of Step S18 from the storage unit 82 (Step S19). The controller 83 inputs the sensor data for the cases C1 to C13 selected in the processing of Step S18 to the transformer whose data was acquired in the processing of Step S19. The controller 83 inputs the sensor data to the transformer and acquires from the transformer time-series data of the estimated values of the posture angles of the body parts across the user's entire body and time-series data of the movement velocity of the user's waist (Step S20). The controller 83 generates a gait model (Step S21) based on the time-series data of the estimated values of the posture angles of the body parts across the user's entire body and the time-series data of the movement velocity of the user's waist acquired in the processing of Step S20. The controller 83 transmits the data of the gait model generated in the processing of Step S21 to the electronic device 20 via the network 2 by using the communication unit 81 (Step S22).

In the electronic device 20, the controller 27 receives the data of the gait model from the server 80 via the network 2 by using the communication unit 21 (Step S23). The controller 27 causes the output unit 24 to output the received data of the gait model (Step S24).

After executing the processing of Step S24, the information processing system 101 terminates the estimation processing. The information processing system 101 may perform the estimation processing again after the estimation processing is completed. In the estimation processing to be performed again, the information processing system 101 may start from the processing of Step S14. The information processing system 101 may repeat the estimation processing until the electronic device 20 receives an input from the input unit 22 instructing termination of the estimation processing. As described above, upon receiving an input instructing termination of the estimation processing, the electronic device 20 may transmit a signal instructing termination of data detection to the multiple sensor devices 10 as a broadcast signal. As described above, upon receiving the signal instructing termination of data detection, each sensor device 10 may terminate the data detection.

The information processing system 101 can achieve the same or similar effects to the information processing system 1.

The present disclosure has been described based on the drawings and examples, but note that a variety of variations and amendments may be easily made by one skilled in the art based on the present disclosure. Therefore, note that such variations and amendments are included within the scope of the present disclosure. For example, the functions and so forth included in each functional part can be rearranged in a logically consistent manner. Multiple functional parts and so forth may be combined into a single part or divided into multiple parts. Further, each embodiment according to the present disclosure described above does not need to be implemented exactly as described in the embodiment, and may be implemented with features having been combined or omitted as appropriate. A variety of variations and amendments to the content of the present disclosure can be made by one skilled in the art based on the present disclosure. Accordingly, such variations and amendments are included in the scope of the present disclosure. For example, in each embodiment, each functional part, each means, each step and so on can be added to other embodiments so long as there are no logical inconsistencies, or can be replaced with each functional part, each means, each step, and so on of other embodiments. In each embodiment, a plurality of each functional part, each means, each step, and so on can be combined into a single functional part, means, or step or divided into multiple functional parts, means, or steps. Each of the above-described embodiments of the present disclosure is not limited to faithful implementation of each of the described embodiments, and may be implemented by combining or omitting some of the features as appropriate.

For example, the electronic device 20 or the server 80 may include a filter that can be applied to data output from the trained model. The filter is, for example, a Butterworth filter.

For example, in the above-described embodiment, the periodic exercise was described as walking. In addition, the trained model was described as being trained to output estimated values of the posture angles of the user's body parts while walking. However, the periodic exercise is not limited to walking. The information processing system of the present disclosure is capable of acquiring estimated values of the posture angles of the user's body parts during any periodic exercise. In other words, the trained model can be trained to output estimated values of the posture angles of the body parts when the user is performing any periodic exercise.

For example, in the embodiment described above, the information processing system 1 or 101 was described as estimating the posture angles of a user who walks as exercise in his or her daily life. However, applications of the information processing system of the present disclosure are not limited to this application.

As another example of an application, the information processing system of the present disclosure may be used to allow other users to watch the way in which a user walks at an event venue. In this case, in the information processing system 1 illustrated in FIG. 3, the controller 27 of the electronic device 20 may transmit the generated gait model data to a projection device at an event venue as an external device via the network 2 or near-field wireless communication using the communication unit 21. In the information processing system 101 illustrated in FIG. 39, the controller 83 of the server 80 may transmit the generated gait model data to a projection device at an event venue as an external device via the network 2 using the communication unit 21. With this configuration, the projection device at the event venue can project the gait model representing the way in which a user walks onto a screen or the like.

As yet another example of an application, the information processing system of the present disclosure may be used to generate images and so forth of the way in which a character walks in a movie or game, etc., using the generated gait model. By attaching the sensor devices 10 to a variety of users, a variety of gait models can be generated.

For example, the communication unit 11 of the sensor device 10 may further include at least one communication module that can connect to the network 2 as illustrated in FIG. 39. The communication module is, for example, a communication module compatible with mobile communication standards such as LTE, 4G, or 5G. In this case, in the information processing system 101 as illustrated in FIG. 39, the controller 16 of the sensor device 10 may directly transmit the data detected by the sensor device 10 to the server 80 via the network 2 by using the communication unit 11.

For example, in the embodiment described above, the cases C5 to C8, C12, and C13 are described as including sensor data representing movement of the user's wrist. However, in the cases C5 to C8, C12, and C13, instead of sensor data representing the movement of the user's wrist, sensor data representing the movement of a part of the user's forearm other than the wrist may be used.

For example, in the embodiment described above, the sensor device 10 is described as including the communication unit 11 as illustrated in FIG. 3 and FIG. 39. However, the sensor device 10 does not need to include the communication unit 11. In this case, sensor data detected by the sensor device 10 may be transferred, via a storage medium such as an SD (Secure Digital) memory card, to a device such as the electronic device 20 or the server 80 that will estimate the posture angles. SD memory cards are also called "SD cards". The sensor device 10 may be configured to allow insertion of a storage medium such as an SD memory card.

For example, an embodiment in which a general-purpose computer is made to function as the electronic device 20 according to this embodiment is also possible. Specifically, a program describing processing content that realizes each function of the electronic device 20 according to this embodiment is stored in the memory of a general-purpose computer, and the program is read out and executed by a processor of the general-purpose computer. Therefore, the configuration according to this embodiment can also be realized as a program executable by a processor or a non-transitory computer-readable medium storing this program.

REFERENCE SIGNS

1, 101 information processing system

2 network

10, 10A, 10B, 10C, 10D, 10D-1, 10D-2, 10E, 10E-1, 10E-2, 10F, 10F-1, 10F-2sensor device

11 communication unit

12 sensor unit

13 notification unit

14 output unit

15 storage unit

16 controller

20 electronic device (information processing device)

21 communication unit

22 input unit

23 notification unit

24 output unit

25 vibration unit

26 storage unit

27 controller

30 transformer

40 encoder

41, 42, 44, 45, 46, 47 functional unit

43 layer

50 decoder

51, 52, 54, 55, 56, 57, 58, 59, 60, 61, 70 functional unit

53 layer

80 server (information processing device)

81 communication unit

82 storage unit

83 controller

C1, C2, C3, C4, C5, C6, C7, C8, C9, C10, C11, C12, C13 case

D10AG, D10AL, D10BL, D10CG, D10CL, D10DL-1, D10DL-2, D10EL-1, D10EL-2, D10FL-1, D10FL-2

**Claims**

1. An information processing device comprising:

   a controller configured to acquire an estimated value of a posture angle of at least any one of multiple body parts of a user based on sensor data representing movement of at least some of the body parts of the user and a trained model,
   wherein the trained model is trained and outputs the estimated value of the posture angle when input with the sensor data.

2. The information processing device according to claim 1,
   wherein the controller is configured to acquire the sensor data from a sensor device worn on at least some body parts of the user.

3. The information processing device according to claim 1 or 2,

wherein the trained model is trained and outputs the estimated value of the posture angle, the posture angle being a posture angle occurring when the user performs periodic exercise.

4. The information processing device according to any one of claims 1 to 3,
wherein the trained model is trained and outputs the estimated value of the posture angle when input with the sensor data, the sensor data representing movement of a head of the user.

5. The information processing device according to any one of claims 1 to 3,
wherein the trained model is trained and outputs the estimated values of the posture angles when input with the sensor data, the sensor data including sensor data representing movement of a head of the user and sensor data representing movement of either one of two ankles of the user.

6. The information processing device according to any one of claims 1 to 3,
wherein the trained model is trained and outputs the estimated values of the posture angles when input with the sensor data, the sensor data including sensor data representing movement of a head of the user and sensor data representing movement of either one of two feet of the user.

7. The information processing device according to any one of claims 1 to 3,
wherein the trained model is trained and outputs the estimated values of the posture angles when input with the sensor data, the sensor data including sensor data representing movement of a head of the user and sensor data representing movement of either one of two thighs of the user.

8. The information processing device according to any one of claims 1 to 3,
wherein the trained model is trained and outputs the estimated values of the posture angles when input with the sensor data, the sensor data including sensor data representing movement of a head of the user and sensor data representing movement of either one of two forearms of the user.

9. The information processing device according to any one of claims 1 to 3,
wherein the trained model is trained and outputs the estimated values of the posture angles when input with the sensor data, the sensor data including sensor data representing movement of a head of the user, sensor data representing movement of either one of two forearms of the user, and sensor data representing movement of either one of two ankles of the user.

10. The information processing device according to any one of claims 1 to 3,
wherein the trained model is trained and outputs the estimated values of the posture angles when input with the sensor data, the sensor data including sensor data representing movement of a head of the user, sensor data representing movement of either one of two forearms of the user, and sensor data representing movement of either one of two feet of the user.

11. The information processing device according to any one of claims 1 to 10,
wherein the trained model is a transformer.

12. The information processing device according to any one of claims 1 to 11,
wherein the trained model is trained and outputs the estimated value of the posture angle, the posture angle being a posture angle of the user while walking.

13. The information processing device according to claim 12,
wherein the controller is configured to generate a gait model representing a way in which the user walks based on the estimated values of the posture angles of the body parts across an entire body of the user and data of a movement velocity of a waist of the user.

14. The information processing device according to claim 13,

wherein the trained model is trained and outputs an estimated value of the movement velocity of the waist of the user when input with the sensor data, and
the controller is configured to acquire the estimated value of the movement velocity of the waist of the user based on the trained model.

**15.** The information processing device according to any one of claims 12 to 14,
wherein the controller is configured to evaluate a gait of the user based on the estimated value of the posture angle.

**16.** The information processing device according to any one of claims 13 to 15, further comprising:

an output unit,
wherein the controller is configured to cause the output unit to output data of the gait model.

**17.** The information processing device according to any one of claims 13 to 15, further comprising:

a communication unit,
wherein the controller is configured to transmit data of the gait model to an external device by using the communication unit.

**18.** An electronic device comprising:
an output unit configured to output data of the gait model generated by the information processing device according to any one of claims 13 to 17.

**19.** An information processing system comprising:

an information processing device configured to acquire an estimated value of a posture angle of at least any one of multiple body parts of a user based on sensor data representing movement of at least some of the body parts of the user and a trained model,
wherein the trained model is trained and outputs the estimated value of the posture angle when input with the sensor data.

**20.** An information processing method comprising:

acquiring an estimated value of a posture angle of at least any one of multiple body parts of a user based on sensor data representing movement of at least some of the body parts of the user and a trained model,
wherein the trained model is trained and outputs the estimated value of the posture angle when input with the sensor data.

**21.** A program configured to cause a computer to execute:

acquiring an estimated value of a posture angle of at least any one of multiple body parts of a user based on sensor data representing movement of at least some of the body parts of the user and a trained model,
wherein the trained model is trained and outputs the estimated value of the posture angle when input with the sensor data.

# FIG. 1

# FIG. 2

LOCAL COORDINATE SYSTEM

GLOBAL COORDINATE SYSTEM

# FIG. 3

SENSOR DEVICE — 10

- NOTIFICATION UNIT — 13
  - OUTPUT UNIT — 14
- COMMUNICATION UNIT — 11
- STORAGE UNIT — 15
- SENSOR UNIT — 12
- CONTROLLER — 16

ELECTRONIC DEVICE — 20

- NOTIFICATION UNIT — 23
  - OUTPUT UNIT — 24
  - VIBRATION UNIT — 25
- COMMUNICATION UNIT — 21
- STORAGE UNIT — 26
- INPUT UNIT — 22
- CONTROLLER — 27

1

EP 4 446 698 A1

## FIG. 4

# FIG. 5

Linear

Concat

Scaled Dot-Product Attention

70

h

Linear

Linear

Linear

V

K

Q

44

# FIG. 6

70

MatMul

Softmax

Mask(opt.)

Scale

MatMul

Q

K

V

# FIG. 7

| | C1 | C2 | C3 | C4 | C5 | C6 | C7 | C8 | C9 | C10 | C11 | C12 | C13 |
|---|---|---|---|---|---|---|---|---|---|---|---|---|---|
| SENSOR DATA D10AG (SENSOR DEVICE 10A) | ✓ | ✓ | ✓ | ✓ | ✓ | ✓ | ✓ | ✓ | | | | | |
| SENSOR DATA D10AL (SENSOR DEVICE 10A) | ✓ | ✓ | ✓ | ✓ | ✓ | ✓ | ✓ | ✓ | | | | | |
| SENSOR DATA D10BL (SENSOR DEVICE 10B) | | | | | ✓ | ✓ | ✓ | ✓ | | | | ✓ | ✓ |
| SENSOR DATA D10EL-1 OR D10EL-2 (SENSOR DEVICE 10E-1 OR 10E-2) | | ✓ | | | | ✓ | | | | | | | |
| SENSOR DATA D10FL-1 OR D10FL-2 (SENSOR DEVICE 10F-1 OR 10F-2) | | | ✓ | | | | ✓ | | | | | | |
| SENSOR DATA D10FL-1 AND D10FL-2 (SENSOR DEVICE 10F-1 AND 10F-2) | | | | | | | | ✓ | ✓ | | | | ✓ |
| SENSOR DATA D10DL-1 OR D10DL-2 (SENSOR DEVICE 10D-1 OR 10D-2) | | | | ✓ | | | | | | | | | |
| SENSOR DATA D10DL-1 AND D10DL-2 (SENSOR DEVICE 10D-1 AND 10D-2) | | | | | | | | | | ✓ | | | |
| SENSOR DATA D10CG (SENSOR DEVICE 10C) | | | | | | | | | | | ✓ | ✓ | ✓ |
| SENSOR DATA D10CL (SENSOR DEVICE 10C) | | | | | | | | | | | ✓ | ✓ | ✓ |

EP 4 446 698 A1

EP 4 446 698 A1

# FIG. 8

# FIG. 9

| SUBJECT | SEX | AGE | HEIGHT [cm] | WEIGHT [kg] | PHYSICAL CHARACTERISTICS |
|---------|--------|-----|-------------|-------------|--------------------------|
| SU1 | MALE | 33 | 171 | 100 | HEAVY WEIGHT, MALE |
| SU2 | FEMALE | 70 | 151 | 39 | LIGHT WEIGHT, FEMALE |
| SU3 | FEMALE | 38 | 155 | 41 | LIGHT WEIGHT, YOUNG, FEMALE |
| SU4 | FEMALE | 65 | 149 | 70 | HEAVY WEIGHT, FEMALE |
| SU5 | MALE | 22 | 163 | 65 | NORMAL HEIGHT AND WEIGHT, MALE |
| SU6 | FEMALE | 66 | 149 | 47 | SHORT, FEMALE |
| SU7 | FEMALE | 65 | 148 | 47 | SHORT, FEMALE |
| SU8 | MALE | 57 | 178 | 81 | TALL, MALE |

EP 4 446 698 A1

## FIG. 10

SUBJECT SU6 (CASE C6, NECK)

## FIG. 11

SUBJECT SU6 (CASE C6, CHEST)

## FIG. 12

SUBJECT SU6 (CASE C6, RIGHT UPPER ARM)

## FIG. 13

SUBJECT SU6 (CASE C6, LEFT UPPER ARM)

FIG. 14

SUBJECT SU6 (CASE C6, RIGHT FOREARM)

FIG. 15

SUBJECT SU6 (CASE C6, LEFT FOREARM)

## FIG. 16

SUBJECT SU6 (CASE C6, RIGHT THIGH)

## FIG. 17

SUBJECT SU6 (CASE C6, LEFT THIGH)

## FIG. 18

SUBJECT SU6 (CASE C6, RIGHT LOWER LEG)

## FIG. 19

SUBJECT SU6 (CASE C6, LEFT LOWER LEG)

FIG. 20

SUBJECT SU6 (CASE C6, RIGHT FOOT)

FIG. 21

SUBJECT SU6 (CASE C6, LEFT FOOT)

# FIG. 22

**SUBJECT SU7 (CASE C6, RIGHT THIGH)**

# FIG. 23

**SUBJECT SU1 (CASE C6, RIGHT THIGH)**

# FIG. 24

SUBJECT SU3 (CASE C6, RIGHT THIGH)

# FIG. 25

SUBJECT SU6 (CASE C6, RIGHT THIGH)

## FIG. 26

SUBJECT SU5 (CASE C6, RIGHT THIGH)

## FIG. 27

SUBJECT SU2 (CASE C6, RIGHT THIGH)

## FIG. 28

SUBJECT SU4 (CASE C6, RIGHT THIGH)

## FIG. 29

SUBJECT SU8 (CASE C6, RIGHT THIGH)

## FIG. 30

**SUBJECT SU7 (CASE C6, RIGHT UPPER ARM)**

## FIG. 31

**SUBJECT SU1 (CASE C6, RIGHT UPPER ARM)**

FIG. 32

SUBJECT SU3 (CASE C6, RIGHT UPPER ARM)

FIG. 33

SUBJECT SU6 (CASE C6, RIGHT UPPER ARM)

## FIG. 34

SUBJECT SU5 (CASE C6, RIGHT UPPER ARM)

## FIG. 35

SUBJECT SU2 (CASE C6, RIGHT UPPER ARM)

## FIG. 36

SUBJECT SU4 (CASE C6, RIGHT UPPER ARM)

## FIG. 37

SUBJECT SU8 (CASE C6, RIGHT UPPER ARM)

# FIG. 38

```
              ┌─────────────┐
              │    START    │
              └─────────────┘
                     │
                     ▼
      ┌──────────────────────────────┐
      │        ACCEPT INPUT          │─── S1
      └──────────────────────────────┘
                     │
                     ▼
      ┌──────────────────────────────┐
      │   TRANSMIT BROADCAST SIGNAL   │─── S2
      └──────────────────────────────┘
                     │
                     ▼
      ┌──────────────────────────────┐
      │      RECEIVE SENSOR DATA       │─── S3
      └──────────────────────────────┘
                     │
                     ▼
      ┌──────────────────────────────┐
      │  SELECT ANY ONE OF CASES C1 TO C3 │─── S4
      └──────────────────────────────┘
                     │
                     ▼
      ┌──────────────────────────────┐
      │  ACQUIRE DATA OF TRANSFORMER   │─── S5
      └──────────────────────────────┘
                     │
                     ▼
      ┌──────────────────────────────┐
      │  ACQUIRE ESTIMATED VALUES OF   │─── S6
      │     POSTURE ANGLES ETC.        │
      └──────────────────────────────┘
                     │
                     ▼
      ┌──────────────────────────────┐
      │     GENERATE GAIT MODEL        │─── S7
      └──────────────────────────────┘
                     │
                     ▼
      ┌──────────────────────────────┐
      │      OUTPUT GAIT MODEL         │─── S8
      └──────────────────────────────┘
                     │
                     ▼
              ┌─────────────┐
              │     END     │
              └─────────────┘
```

## FIG. 39

SERVER 80

COMMUNICATION UNIT 81

STORAGE UNIT 82

CONTROLLER 83

2

101

SENSOR DEVICE 10

NOTIFICATION UNIT 13

OUTPUT UNIT 14

COMMUNICATION UNIT 11

STORAGE UNIT 15

SENSOR UNIT 12

CONTROLLER 16

ELECTRONIC DEVICE 20

NOTIFICATION UNIT 23

OUTPUT UNIT 24

COMMUNICATION UNIT 21

VIBRATION UNIT 25

INPUT UNIT 22

CONTROLLER 27

EP 4 446 698 A1

FIG. 40

| SENSOR DEVICE ~10 | ELECTRONIC DEVICE ~20 | SERVER ~40 |
|---|---|---|
| | ACCEPT INPUT ~S11 | |
| | TRANSMIT BROADCAST SIGNAL ~S12 | |
| S13~ RECEIVE BROADCAST SIGNAL | | |
| S14~ TRANSMIT SENSOR DATA | | |
| | RECEIVE SENSOR DATA ~S15 | |
| | S16~ TRANSMIT SENSOR DATA | |
| | | RECEIVE SENSOR DATA ~S17 |
| | | SELECT ANY OF CASES C1 TO C3 ~S18 |
| | | ACQUIRE DATA OF TRANSFORMER ~S19 |
| | | ACQUIRE ESTIMATED VALUES OF POSTURE ANGLES ETC. ~S20 |
| | | GENERATE GAIT MODEL ~S21 |
| | | TRANSMIT DATA OF GAIT MODEL ~S22 |
| | RECEIVE DATA OF GAIT MODEL ~S23 | |
| | OUTPUT GAIT MODEL ~S24 | |

<table>
<tr><td colspan="2"><strong>INTERNATIONAL SEARCH REPORT</strong></td><td>International application No.<br><strong>PCT/JP2022/045370</strong></td></tr>
</table>

**A. CLASSIFICATION OF SUBJECT MATTER**

***G01B 21/00***(2006.01)i; ***G01B 21/22***(2006.01)i
FI: G01B21/22; G01B21/00 E

According to International Patent Classification (IPC) or to both national classification and IPC

**B. FIELDS SEARCHED**

Minimum documentation searched (classification system followed by classification symbols)

G01B21/00; G01B21/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/203188 A1 (SONY CORP.) 24 October 2019 (2019-10-24)<br>specification, paragraphs [0001]-[0003], [0020]-[0025], [0049]-[0061], [0065]-[0077],<br>fig. 1, 10-12, 14 | 1-21 |
| Y | JP 2021-100453 A (BRIDGESTONE SPORTS CO., LTD.) 08 July 2021 (2021-07-08)<br>paragraphs [0001], [0002], [0022]-[0053], fig. 1-4 | 1-21 |
| A | JP 9-229667 A (IMEEJI JOHO KAGAKU KENKYUSHO) 05 September 1997 (1997-09-05)<br>entire text, all drawings | 1-21 |
| A | JP 2008-289866 A (XSENS TECHNOLOGIES BV) 04 December 2008 (2008-12-04)<br>entire text, all drawings | 1-21 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 February 2023** | **21 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/045370**

| Patent document cited in search report | | | Publication date (day/month/year) | Patent family member(s) | | | Publication date (day/month/year) |
|---|---|---|---|---|---|---|---|
| WO | 2019/203188 | A1 | 24 October 2019 | US | 2021/0141443 | A1 | |
| | | | | paragraphs [0001]-[0003], [0067]-[0073], [0112]-[0124], fig. 1, 10-12, 14 | | | |
| | | | | EP | 3782548 | A1 | |
| | | | | CN | 111954491 | A | |
| JP | 2021-100453 | A | 08 July 2021 | (Family: none) | | | |
| JP | 9-229667 | A | 05 September 1997 | (Family: none) | | | |
| JP | 2008-289866 | A | 04 December 2008 | US | 2008/0262772 | A1 | |
| | | | | whole document | | | |
| | | | | EP | 1970005 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021201275 A

- JP 2020201183 A

**Non-patent literature cited in the description**

- **ASHISH VASWANI et al.** Attention Is All You Need. *arXiv:1706.03762v5,* 12 June 2017

- **Y KOBAYASHI ; N. HIDA ; K. NAKAJIMA ; M. FUJIMOTO ; M. MOCHIMARU.** *2019: AIST Gait Database 2019,* 11 November 2021, https ://unit.aist.go.jp/harc/ExPART/GDB2019_e.ht ml>